# EUROPEAN PATENT SPECIFICATION

(11) **EP 0 553 130 B1**
(45) Date of publication and mention of the grant of the patent: **03.01.1996**
(21) Application number: 91917487.0
(22) Date of filing: 13.09.1991
(51) Int. Cl.: C07D 207/38

(54) **PYRROLIDINE DIONE DERIVATIVES**
PYRROLIDIN-DIONDERIVATE
DERIVES DE PYRROLIDINE DIONE

(30) Priority: 15.10.1990 US 597614
(43) Date of publication of application: 04.08.1993
(73) Proprietor: PFIZER INC., New York, N.Y. 10017-5755 (US)
(72) Inventor: MYLARI, Banavara, L., Waterford, CT 06385 (US)
(74) Representative: Moore, James William, Dr.
(86) International application number: US9106483
(87) International publication number: WO9206954

(56) References cited:
- J. Pharm. Pharmacol., vol. 17, 1965, A.H. Beckett et al: "Some pyrroline derivatives as potential antiinflammatory agents", pages 498-503, see compounds II
- Chemical Abstracts, vol. 89, no. 7, 14 August 1978, (Columbus, Ohio, US) Sato Yasunobu et al: "Synthetic studies on cardiovascular agents. IV. Synthesis of fused dihydropyridine derivatives", page 570, abstract 59846w, & Yakugaku Zasshi 1978, 98(4), 448-65, see reg. no. 67044-08-0, 67044-09-1, 67044-10-4, 67044-11-5, 67044-12-6, 67044-13-7 and 67044-14-8
- Tetrahedron, vol. 40, no. 21, 1984, J.E. Baldwin et al.: "Gamma-lactam analogues of penicillanic and carbapenicillanic acids", pages 4513-4525, see compound 246
- J. Med. Chem., vol. 34, no. 3, March 1991, B.L. Mylari et al.: "A highly specific aldose reductase inhibitor, ethyl 1-benzyl-3-hydroxy-2(5H)-oxo- pyrrole-4-carboxylate, and its congeners", pages 1011-1018, see the whole article

## Description

### BACKGROUND OF THE INVENTION

This invention relates to pyrrolidine dione esters and carboxamides that are inhibitors of the enzyme aldose reductase. These compounds are useful in the treatment and prevention of diabetes associated complications such as neuropathy, nephropathy, retinopathy and cataracts.

Southwick et al., J. Am. Chem. Soc., 75, 3413 (1953), refer to the pyrrolidine dione ester ethyl 1-benzyl-4,5-dioxopyrrolidine-3-carboxylate, without referring to aldose reductase inhibitory activity. Kadin, Saul B., J. Med. Chem., 19, 172 (1976), refers to certain carboxamides having N-alkyl side chains, also without referring to aldose reductase inhibitory activity.

### Summary of the Invention

This invention relates to compounds of the formula 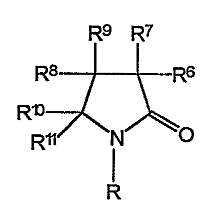 wherein either
(1) R is 9-florenyl or aralkyl wherein the alkyl moiety contains one or two carbon atoms and wherein the aryl moiety is an aromatic or heteroaromatic ring selected from phenyl, furyl, thienyl, pyridyl and naphthyl, and said aromatic or heteroaromatic ring is optionally substituted with one or two substituents independently selected from bromine, chlorine, fluorine, (C₁-C₃) alkyl and (C₁-C₃) alkoxy;
   R⁶ and R⁷ together form an oxo group;
   R⁸ is hydrogen;
   R⁹ is COR¹;
   R¹ is OR or NHR³, wherein R is (C₁-C₃) alkyl and R³ is selected from furyl, thienyl, 2-thiazolyl, 2-benzothiazolyl, 3-methyl-5-isothiazolyl and 5-phenyl-1,3,4-thiazolyl;
and R¹⁰ and R¹¹ are each hydrogen; or
(2) R is as defined above;
   R⁶ is hydrogen;
   R⁷ is CONHR³ wherein R³ is as defined above;
   R⁸ and R⁹ together form an oxo group; and
   R¹⁰ and R¹¹ are each hydrogen; or
(3) R is hydrogen;
   R⁶ and R⁷ together form an oxo group;
   R⁸ is hydrogen;
   R⁹ is COR¹ wherein R¹ is as defined above;
   R¹⁰ and R¹¹ together with the carbon atom to which they are attached form an
optionally substituted, spiro-fused bicyclic system of the formula: 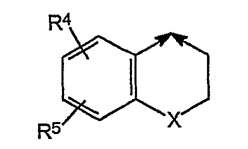
wherein X is methylene, oxygen or sulphur and R⁴ and R⁵ are independently
selected from hydrogen,bromine, chlorine, fluorine and (C-C₃)alkoxy; with the proviso that when R¹ is ethoxy or methoxy, R is other than benzyl or substituted benzyl, wherein the substituents are 2-, 3-, or 4-chloro, 4-methoxy or 3,4-dimethoxy groups; phenethyl or substituted phenethyl wherein the substituents are 4-chloro or 4-methyl;
and the pharmaceutically acceptable salts thereof.

Thus in its different aspects the invention covers compounds having the formulae: 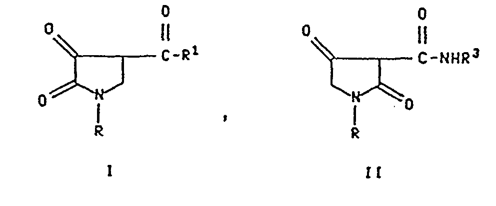 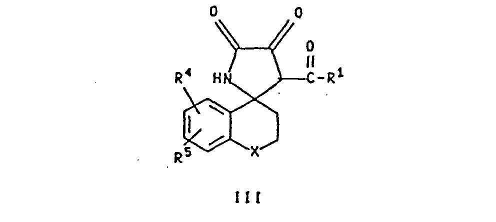 wherein the substituent groups R, R¹, R³, R⁴, R⁵ and X are as defined above.

The compounds of the formula III contain chiral centers and therefore may exist in different stereochemical forms. The compounds of the formulae I and II may contain chiral centers depending on the value of R and therefore may exist in different stereochemical forms. The present invention includes all stereoisomers of the compounds having formulae I, II and III.

The compounds having formulae I, II and III may also exist in their corresponding enolic forms. For example, compounds of the formula I may also exist in the corresponding enolic form represented by the formula

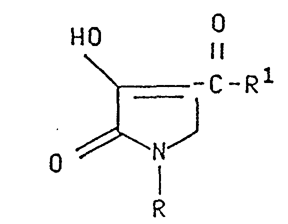

The present invention includes all enolic forms of the compounds having formula I, II or III.

The present invention also relates to a pharmaceutical composition for preventing or alleviating a diabetes associated complication such as neuropathy, nephropathy, retinopathy and cataracts in a diabetic mammal, including a human, comprising an amount of a compound having formula I, II or III effective in preventing or alleviating such complication, and a pharmaceutically acceptable carrier.

Preferred compounds of this invention are those having the formula I wherein R¹ is ethoxy and R is 9-florenyl. More preferred compounds are those having the formula I wherein R¹ is ethoxy and R is aralkyl wherein the alkyl moiety is methylene and the aryl moiety is selected from phenyl, naphthyl, 2-thienyl, 2-pyridyl, 2-furyl, 4-bromophenyl and 3,4-dichlorophenyl.

Other preferred compounds of this invention are those having the formula II wherein R is aralkyl wherein the alkyl moiety is methylene and the aryl moiety is selected from phenyl, 3,4-dichlorophenyl and 4-methoxyphenyl, and R¹ is selected from 3-methylisothiazol-5-yl, benzothiazol-2-yl, 5-methylisothiazol-3-yl, thiazol-2-yl and 3-phenyl-1,2,4-thiazol-5-yl.

Other preferred compounds of this invention are those having the formula III wherein R⁴ is 6-fluoro, R⁵ is hydrogen, X is oxygen and R¹ is ethoxy.

Specific preferred compounds of this invention include the following:
ethyl 1-(thien-2-ylmethyl)-4,5-dioxopyrrolidine-3-carboxylate;
ethyl 1-(pyrid-2-ylmethyl)-4,5-dioxopyrrolidine-3-carboxylate;
ethyl 1-(pyrid-4-ylmethyl)-4,5-dioxopyrrolidine-3-carboxylate;
ethyl 1-(naphth-1-ylmethyl)-4,5-dioxopyrrolidine-3-carboxylate;
ethyl 1-(4-bromobenzyl)-4,5-dioxopyrrolidine-3-carboxylate;
ethyl 1-(furan-2-ylmethyl)-4,5-dioxopyrrolidine-3-carboxylate;
ethyl 1-(3,4-dichlorobenzyl)-4,5-dioxopyrrolidine-3-carboxylate;
propyl 1-(benzyl)-4,5-dioxopyrrolidine-3-carboxylate;
ethyl 1-(9-florenyl)-4,5-dioxopyrrolidine-3-carboxylate;
N-(3-methylisothiazol-5-yl)-1-benzyl-4,5-dioxopyrrolidine-3-carboxamide;
N-(benzothiazol-2-yl)-1-benzyl-4,5-dioxopyrrolidine-3-carboxamide;
N-(5-methyl-isoxazol-3-yl)-1-benzyl-4,5-dioxopyrrolidine-3-carboxamide;
N-(thiazole-2-yl)-1-(3,4-dichlorobenzyl)-2,4-dioxopyrrolidine-3-carboxamide;
N-(benzothiazol-2-yl)-1-(3,4-dichlorobenzyl)-2,4-dioxopyrrolidine-3-carboxamide;
N-(3-phenyl-1,2,4-thiadiazol-5-yl)-1-(3,4-dichlorobenzyl)-2,4-dioxopyrrolidine-3-carboxamide;
N-(benzothiazol-2-yl)-1-(2-phenylethyl)-2,4-dioxopyrrolidine-3-carboxamide;
N-(3-methyl-isothiazol-5-yl)-1-(4-methoxybenzyl)-2,4-dioxopyrrolidine-3-carboxamide;
N-(benzothiazol-2-yl)-1-(4-methoxybenzyl)-2,4-dioxopyrrolidine-3-carboxamide; and
spiro [4H-1-benzopyran-4,2'-pyrrolidine]3'-carboxylic acid, 6-fluoro-2,3-dihydro-4',5'-dioxo-, ethyl ester.

Other compounds of this invention include the following:
ethyl 1-(4-fluorobenzyl)-4,5-dioxopyrridine-3-carboxylate;
ethyl 1-(3,4-difluorobenzyl)-4,5-dioxopyrrolidine-3-carboxylate;
ethyl 1-(3,4-difluorothien-2-ylmethyl)-4,5-dioxopyrrolidine-3-carboxylate;
ethyl 1-(3,4-difluoropyrid-2-ylmethyl)-4,5-dioxopyrrolidine-3-carboxylate;
methyl 1-(3,4-dimethoxybenzyl)-4,5-dioxopyrrolidine-3-carboxylate;
ethyl 1-(2-fluorofloren-9-yl)-4,5-dioxopyrrolidine-3-carboxylate;
ethyl 1-(2,6-difluorofloren-9-yl)-4,5-dioxopyrrolidine-3-carboxylate;
N-(3-methylisothiazol-5-yl)-1-4-fluorobenzyl-4,5-dioxopyrrolidine-3-carboxamide; and
N-(benzothiazol-2-yl)-1-(3,4-difluorothien-2-yl methyl)-4,5-dioxopyrrolidine-3-carboxamide.

### Detailed Description of the Invention

The preparation of compounds having formulae I, II and III are described in the reaction schemes and discussion that follow. In the following reaction schemes and discussion, unless otherwise stated, R¹, R, R³, R⁴, R⁵ and X are defined as above.

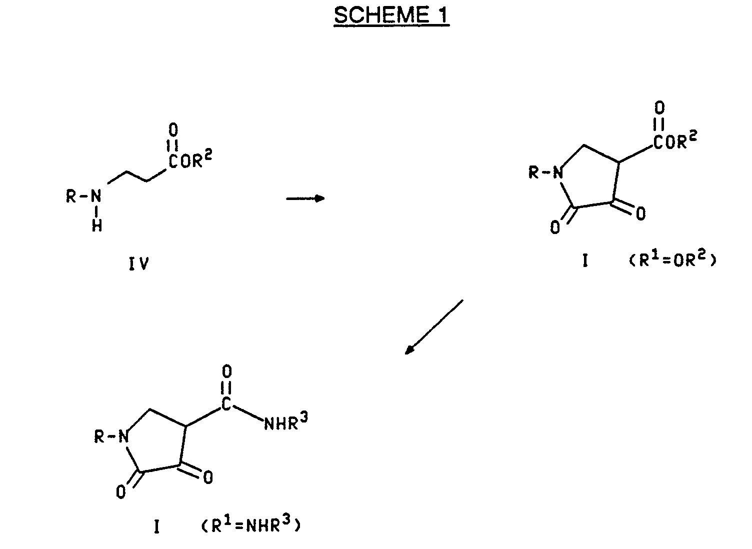

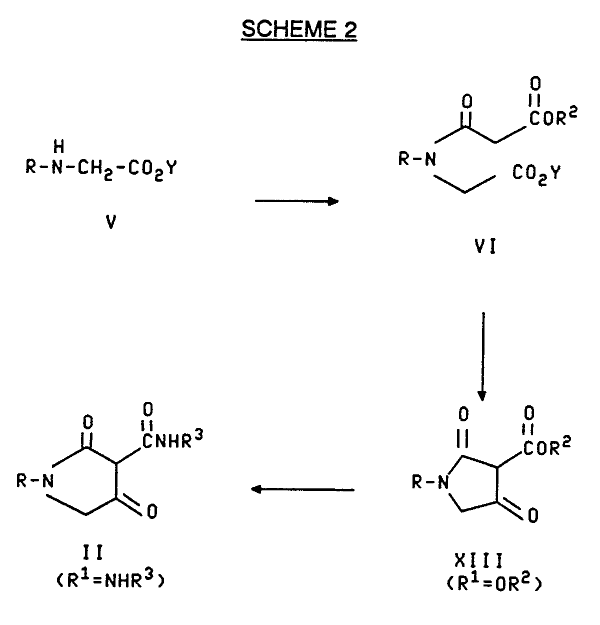

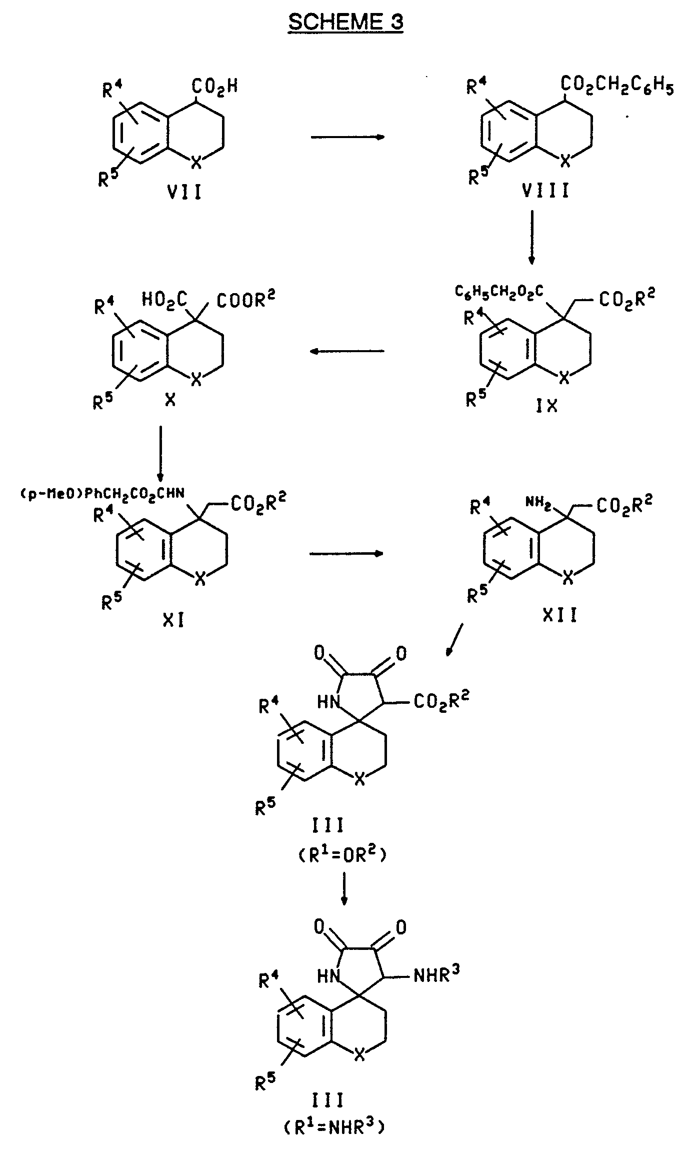

Scheme 1 illustrates a method of preparing compounds of the formula I. Referring to scheme 1, the starting material having formula IV is prepared by reacting an aralkylamine of the formula RNH₂ with an alkyl acrylate of the formula 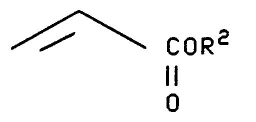 This reaction is generally conducted in dimethylformamide (DMF) or an alcoholic (e.g., ethanol) or ether (e.g., diglyme) solvent, at a temperature from about 0°C to about room temperature. Preferably, the reaction is conducted at room temperature in methanol or ethanol. Reaction is usually complete within twelve hours.

Compounds of the formula I wherein R¹ is OR may be prepared from compounds of the formula IV by reacting them with the appropriate dialkyl oxalate of the formula 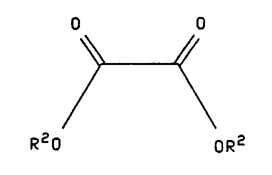 Typically, this reaction is carried out in an alcoholic solvent in the presence of an alkali metal alkoxide or alkaline earth metal alkoxide at a temperature from about 0°C to about 60°C. Preferably, the reaction is carried out at room temperature. Suitable alkali and alkaline earth metal alkoxides include sodium, potassium and calcium ethoxides. The preferred metal is sodium. The preferred alkoxide and alcoholic solvent depends upon the number of carbon atoms in R. Using an alkoxide and alcohol having the same number of carbons in the alkyl portion as R increases the yield.

The compounds of formula I wherein R¹ is OR may be converted to the corresponding compounds of formula I wherein R¹ is NHR³ by heating them in the presence of the appropriate amine of the formula R³NH₂. This reaction is usually conducted in an aromatic hydrocarbon solvent such as benzene, toluene or xylenes. Toluene and xylenes are preferred. Suitable reaction temperatures range from about 50°C to about the reflux temperature of the solvent, with the reflux temperature being preferred.

Scheme 2 illustrates a method of preparing compounds having the formula II. The first two reactions illustrated in scheme 2 (i.e., the preparation of compounds having the formulae VI and XIII) and the preparation of the starting material of formula V are described in J. Chem. Soc., 2121 (1972).

Referring to scheme 2, the starting material of formula V may be prepared by reacting an aralkyl amine of the formula RNH₂ with a compound of the formula L-CH₂-CO₂Y, wherein L is bromine, chlorine or iodine and Y is (C₁-C₃)alkyl. Suitable solvents for this reaction include ethers such as ethyl ether and diglyme and halocarbons such as methylene chloride and chloroform. This reaction is generally conducted in the presence of a tertiary amine such as pyridine, quinoline or a trialkylamine having from one to six carbons in each the alkyl moieties. The preferred solvent is ethyl ether and the preferred amine is triethylamine. The reaction temperature may range from about 0°C to about room temperature.

The compounds of formula V so formed may be converted to the corresponding compounds of the formula VI wherein Y is defined as above, by reacting them with a malonyl chloride of the formula 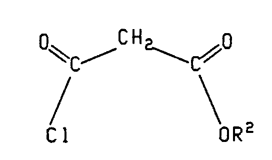 in a halocarbon solvent such as methylene chloride or chloroform, preferably methylene chloride, at a temperature from about 0°C to about 50°C, preferably at about room temperature.

Cyclization of the resulting compounds of formula VI using alkali metal alkoxides or alkaline earth metal alkoxides yields the corresponding compounds of the formula XIII wherein R¹ is OR. The cyclization is usually conducted in an aromatic hydrocarbon solvent such as benzene, toluene or xylenes, preferably benzene, at a temperature from about room temperature to about 120°C, preferably from about 50°C to about 100°C. Suitable metal alkoxides include sodium, potassium and calcium alkoxides. Sodium is the preferred metal. Choosing an alkoxide having the same number of carbons in the alkyl portion as R increases the yield.

Compounds of the formula XIII may be converted into compounds of the formula II by heating them in the presence of the appropriate amine of the formula R³NH₂. This reaction is generally conducted in an aromatic hydrocarbon solvent such as benzene, toluene or xylenes, preferably in toluene or xylenes. Reaction temperatures may range from about 80°C to about the reflux temperature of the solvent. It is preferable to conduct the reaction at the reflux temperature of the solvent.

Scheme III illustrates a method of preparing compounds having the formula III. Referring to scheme 3, the starting material of formula VII may be prepared according to the procedure described in Synthesis, 979 (1979). These compounds are then converted into their corresponding sodium or potassium salts by reaction with aqueous sodium or potassium bicarbonate by methods that are known in the art. These salts are then reacted with a compound of the formula PhCH₂U (Ph=phenyl) wherein U is bromo, chloro or iodo, to obtain the corresponding compounds of formula VIII. Preferably, PhCH₂U is benzyl bromide. This reaction is typically conducted in a halocarbon solvent such as methylene chloride, chloroform or dichloroethane at a temperature from about 20°C to about 60°C. The preferred solvent is methylene chloride and the preferred temperature is about 30°C.

Compounds of the formula IX may be formed by reacting the corresponding compounds of the formula VIII from the foregoing reaction with a compound of the formula WCH₂CO₂R, wherein W is bromo, chloro or iodo, in the presence of an alkali metal hydride. Suitable solvents for this reaction include dimethylformamide (DMF) and ethers such as diglyme. The reaction is generally conducted at a temperature from about 0°C to about 60°C. It is preferably conducted in the presence of sodium hydride at room temperature in DMF.

Hydrogenation of the compounds having formula IX yields the corresponding compounds of the formula X. The hydrogenation is usually performed at a pressure from about 0.5 to about 5 atmospheres, preferably at about 3.0 atmospheres, in an alcoholic solvent such as methanol or ethanol, preferably methanol. Other solvents which may be used in the practice of the invention include cyclic ethers, such as tetrahydrofuran and dioxane, and esters of (C₁-C₆) alkanoic acids and (C₁-C₆) alkyl alcohols, such as ethyl acetate. The temperature may range from about 10°C to about 50°C, and is preferably about room temperature.

The compounds of formula X so formed may be converted to the corresponding compounds of the formula XI by reacting them first with diphenylphosphoryl azide and then with benzyl or para-methoxybenzyl alcohol, preferably para-methoxybenzyl alcohol. Typically, these reactions are carried out at a temperature from about 0°C to about 60°C, preferably at about 30°C. Suitable solvents for these reactions include benzene, toluene and xylenes. Benzene is preferred.

Compounds of the formula XII may be prepared by hydrogenating the corresponding compounds of the formula XI as described above for the hydrogenation of compounds having formula IX. Compounds of the formula III wherein R¹ =COR may be prepared from the corresponding compounds of the formula XII by the procedure illustrated in scheme 1 and described above for preparing compounds of the formula I wherein R¹ is OR from compounds of the formula IV. Compounds of the formula III wherein R1 is NHR³ may be prepared from the corresponding compounds of the formula III wherein R¹ is OR by the procedure illustrated in scheme 1 and described above for preparing compounds of the formula I wherein R¹ is NHR³ from corresponding compounds of the formula I wherein R¹ is OR.

In each of the above reactions, unless otherwise stated, pressure is not critical. Pressures from about 0.5 to about 5 atmospheres are generally acceptable, and ambient pressure, i.e. about 1 atmosphere, is preferred as a matter of convenience.

The compounds of the formulae I, and II and their pharmaceutically acceptable salts (hereinafter referred to as the "active compounds") are inhibitors of the enzyme aldose reductase and are useful in prevention and treatment of diabetes associated complications such as neuropathy, nephropathy, retinopathy and cataracts. The activity of these compounds may be determined using the aldose reductase in vitro assay described in J. Med. Chem., 33, 1859-1865 (1990). Their activity may also be determined in vivo using the procedure described in Metab. Clin. Exp., 28, (Suppl. 1), 456-461 (1979).

Pharmaceutically acceptable salts of compounds of the formula I, II or III may be formed by conventional methods. Preferred salts are those formed with bases either by neutralization or by addition (e.g. megluminium salts). Thus, cationic salts may be readily prepared by treating a compound of formula I, II or III with an aqueous solution of a basic salt of the desired pharmaceutically acceptable cation and evaporating the resulting solution to dryness, preferably under reduced pressure. Alternatively, a lower alkanol solution of a compound of the formula I, II or III may be mixed with an alkoxide of the desired cation and the solution subsequently evaporated to dryness. Suitable pharmaceutically acceptable cations for this purpose include, but are not limited to alkali metal cations such as potassium and sodium, alkaline earth metal cations, such as calcium and magnesium, ammonium and substituted ammonium such as lower alkanolammonium. Base addition salts may be formed from pharmaceutically acceptable amines such as N-methylgluca-mine (meglumine) and glucosamine. In general, the sodium and meglumine salts are preferred.

The active compounds of this invention are useful as inhibitors of the enzyme aldose reductase in the treatment of chronic complications of diabetes, such as diabetic cataracts, retinopathy and neuropathy. As used herein, the term "treatment" is meant to include both the prevention and alleviation of such conditions. The compound may be administered to a subject in need of treatment by a variety of conventional routes of administration, including orally, parenterally and topically. In general, these compounds will be administered orally or parenterally at dosages between about 0.5 and 25 mg/kg body weight of the subject to be treated per day, preferably from about 1.0 to 10 mg/kg. Some variation in dosage will necessarily occur depending on the condition of the subject being treated. The person responsible for administration will determine the appropriate dose for the individual subject.

The active compounds of this invention may be administered alone or in combination with pharmaceutically acceptable carriers, in either single or multiple doses. Suitable pharmaceutical carriers include inert solid diluents or fillers, sterile aqueous solutions and various organic solvents. The pharmaceutical compositions formed by combining the active compounds of this invention and pharmaceutically acceptable carriers are then readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the active compounds in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitioneal administration. In this connection, the sterile aqueous media employed are all readily available by standard techniques known to those skilled in the art.

The active compounds may not only be advantageously employed for the preparation of aqueous pharmaceutical compositions for parenteral administration, as described above, but more particularly for the preparation of pharmaceutical compositions suitable for use as ophthalmic solutions. Such ophthalmic solutions are of principal interest for the treatment of diabetic cataracts by topical administration and the treatment of such conditions in this manner is a preferred embodiment of the present invention. Thus, for the treatment of diabetic cataracts the active compounds of this invention are administered to the eye in the form of an ophthalmic preparation prepared in accordance with conventional pharmaceutical practice (see for example, "Remington's Pharmaceutical Sciences", 15th Edition, pages 1488 to 1501 (Mack Publishing Co., Easton, Pa.)). The ophthalmic preparation will contain an active compound in a concentration from about 0.01 to about 1% by weight, preferably from about 0.05 to about 0.5%, in a pharmaceutically acceptable solution, suspension or ointment. Some variation in concentration will necessarily occur depending on the particular compound employed, the condition of the subject to be treated and the like, and the person responsible for treatment will determine the most suitable concentration for the individual subject.

The ophthalmic preparation will preferably be in the form of a sterile aqueous solution containing, if desired, additional ingredients, for example preservatives, buffers, tonicity agents, antioxidants and stabilizers, non-ionic wetting or clarifying agents, viscosity-increasing agents and the like. Suitable preservatives include benzalkonium chloride, benzethonium chloride, chlorobutanol, thimerosal and the like. Suitable buffers include boric acid, sodium and potassium bicarbonate, sodium and potassium borate, sodium and potassium carbonate, sodium acetate, sodium biphosphate and the like, in amounts sufficient to maintain the pH between about 6 to about 8, preferably between about 7 and about 7.5. Suitable tonicity agents are dextran 40, dextran 70, dextrose, glycerin, potassium chloride, propylene glycol, sodium chloride and the like, such that the sodium chloride equivalent of the ophthalmic solution is in the range 0.9 plus or minus 0.2%. Suitable antioxidants and stabilizers include sodium bisulfite, sodium metabisulfite, sodium thiosulfate, thiourea and the like. Suitable wetting and clarifying agents include polysorbate 80, polysorbate 20. Suitable viscosity-increasing agents include dextran 40, dextran 70, gelatin, glycerin, hydroxyethylcellulose, hydroxpropyl methyl cellulose, lanolin, methylcellulose, petrolatum, poly(ethylene glycol), poly(vinyl alcohol), polyvinylpyr-rolidone, carboxymethylcellulose and the like. The ophthalmic preparation will be administered topically to the eye of the subject in need of treatment by conventional methods, for example in the form of drops or by bathing the eye in the ophthalmic solution.

The present invention is illustrated by the following examples. It will be understood, however, that the invention is not limited to the specific details of these examples. Proton nuclear magnetic resonance spectra (¹H NMR) were measured for solutions in deuterochloroform (CDCI₃) and peak positions are expressed in parts per million (ppm) downfield from tetramethylsilane (TMS). The peak shapes are denoted as follows: s, singlet; d, doublet, t, triplet; q, quartet; m, multiplet; br, broad.

The following examples illustrate but do not limit the scope of this invention.

### Example 1

### Ethyl β-(thien-2-ylmethylamino)-propionate

To an ice-cold solution of thien-2-ylmethylamine (16.1 g) in ethanol (30 ml) was added ethyl acrylate (15.5 ml) and the mixture was stirred overnight. Excess ethanol was removed and the residue was diluted with water. Sufficient 20% potassium hydroxide was added to adjust the pH to about 10.0 and then the resulting mixture was extracted with ethyl acetate (2 x 100 ml). The organic extract was dried and evaporated and the residue was distilled to obtain the title compound (yield: 17.0 g; b.p. 126°C at 3mm Hg pressure).

### Example 2

### Ethyl 1-(thien-2-ylmethyl)-4,5-dioxopyrrolidine-3-carboxylate

To a solution of sodium ethoxide, prepared from sodium metal (2.2 g) and ethanol (20 ml), was added a solution of ethyl β-(2-thien-2-ylmethylamino)-propionate (17.0 g) in diethyl oxalate (11.6 g). The reaction mixture was heated on a steam bath for 1 hour, and then cooled to room temperature. The resulting heavy white precipitate was filtered. The precipitate was dissolved in water. Sufficient 10% hydrochloric acid was added to adjust the pH to about 2.0. The resulting solid was collected and crystallized from methanol to obtain the title compound (yield: 4.5 g; m.p. 98-100°C).

The compounds of Examples 3-14 are compounds of the formula I wherein R¹=OR. They were prepared according to the procedure of Example 2.

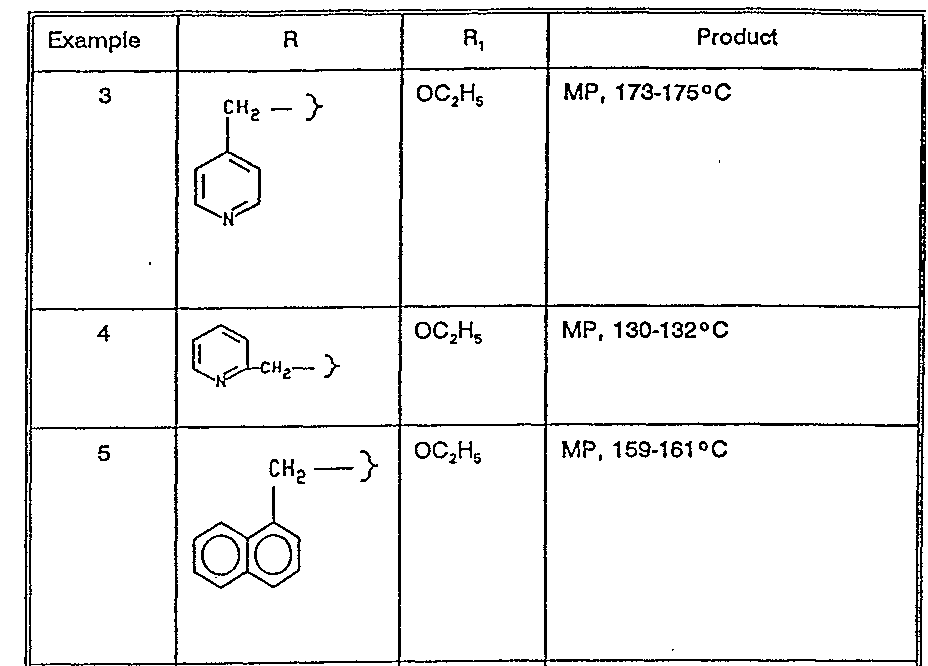

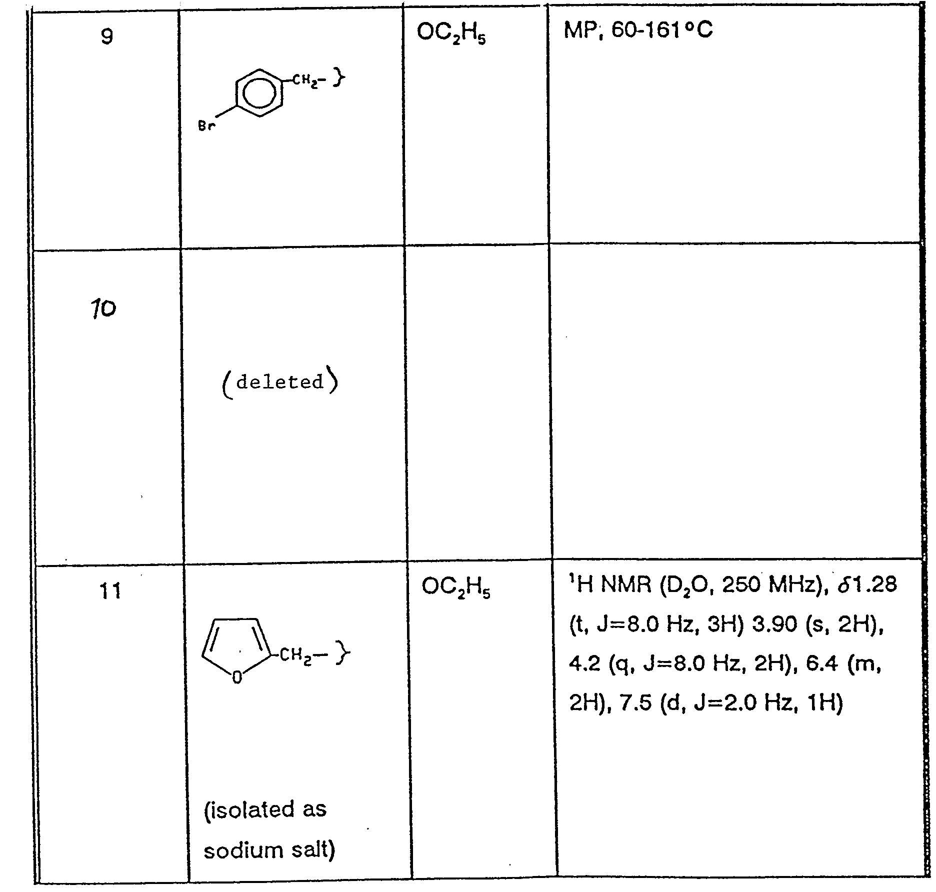

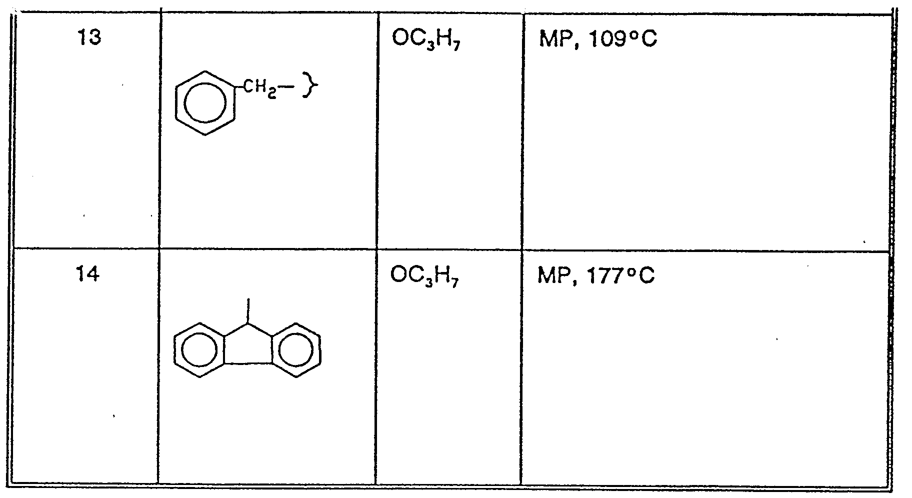

### Example 15

### N-(3-Methylisothiazol-5-yl)-1-benzyl-4,5-dioxopyr-rolidine-3-carboxamide

A mixture of ethyl 1-benzyl-4,5-dioxopyrrolidine-3-carboxylate (1.0 g), 5-amino-3-methylisothiazole (0.60 g) and xylene (15 ml) was refluxed for 1 hour. The precipitated orange solid obtained upon cooling was filtered to obtain the title compound (yield: 0.67 g; m.p. 244-246°C (decomposed upon melting)).

The compounds of Examples 16 and 17 have formula I wherein R¹=NHR³ and were prepared according to the procedure of Example 15.

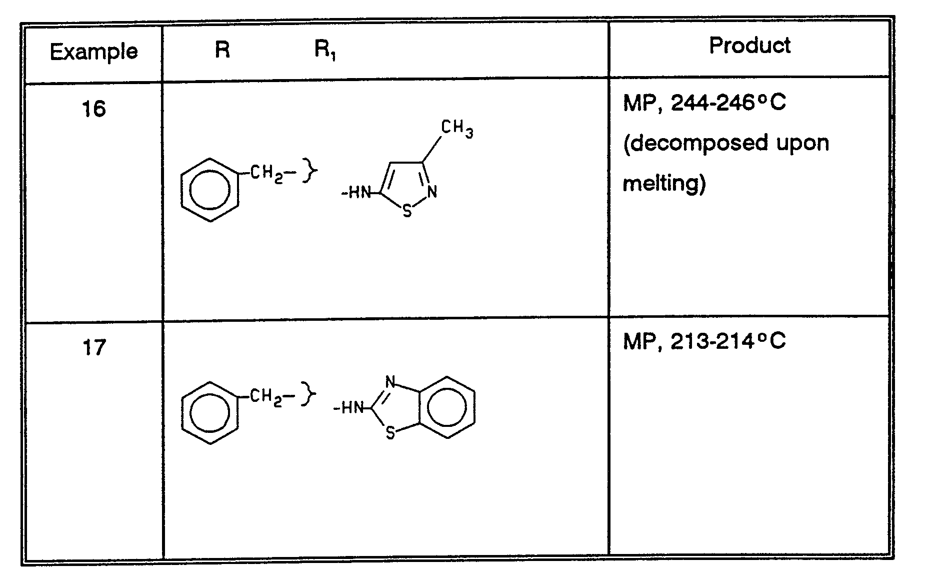

### Example 18

### Ethyl N-(3,4-dichlorobenzyl)glycinate

Triethylamine (27.7 ml) was gradually added to an ice-cold solution of 3,4-dichlorobenzyl amine (35.1 g) in ether (400 ml). To the resulting solution was slowly added ethyl bromoacetate (29.0 ml) over a 1 hour period. After stirring overnight, the reaction mixture was filtered and the filtrate was first concentrated to remove excess ether and then distilled to obtain the title compound (yield: 33.9 g; b.p. 175° at 2mm Hg pressure).

### Example 19

### Ethyl N-(3,4-dichlorobenzyl)-N-(ethoxycarbonylacetyl)-glycinate

To an ice-cold solution of ethyl N-(3,4-dichlorobenzyl) glycinate (33.90 g) in methylene chloride (500 ml) and triethyl amine (17.9 ml) was gradually added a solution of ethyl malonyl monochloride (16.5 ml) in methylene chloride (100 ml). After 5 hours, the reaction mixture was quenched with water (300 ml) and the methylene chloride layer was collected and washed with dilute hydrochloric acid and water. The organic extract was evaporated and the title compound was obtained as an oil (yield: 43.3 g; ¹H NMR (CDCI₃, 60 MHz) δ 1.20 (t, J=7.2 Hz, 6H), 3.60 (s, 2H), 4.2 (q, J=7.2 Hz, 4H), 4.80 (s, 2H) 7.4 (m, 3H)).

### Example 20 (Intermediate)

### Ethyl 1-(3,4-dichlorobenzyl)-2,4-dioxopyrrolidine-3-carboxylate

Sodium ethoxide, prepared by dissolving sodium metal (2.6 g) in ethanol (250 ml), was added to a solution of ethyl N-(3,4-dichlorobenzyl)-N-(ethoxycarbonylacetyl)-glycinate (43.3 g) in benzene (400 ml) and the reaction mixture was refluxed for 3 hours. It was cooled and mixed with ice-water (300 ml). The aqueous layer was collected and sufficient concentrated hydrochloric acid was added to bring the pH to about 2. The precipitated solid was collected and air-dried (yield: 23.3 g; MP, 128-131°C).

The compounds of Examples 21-25 are intermediates for the compounds of formula II and were prepared according to the procedure of Example 20.

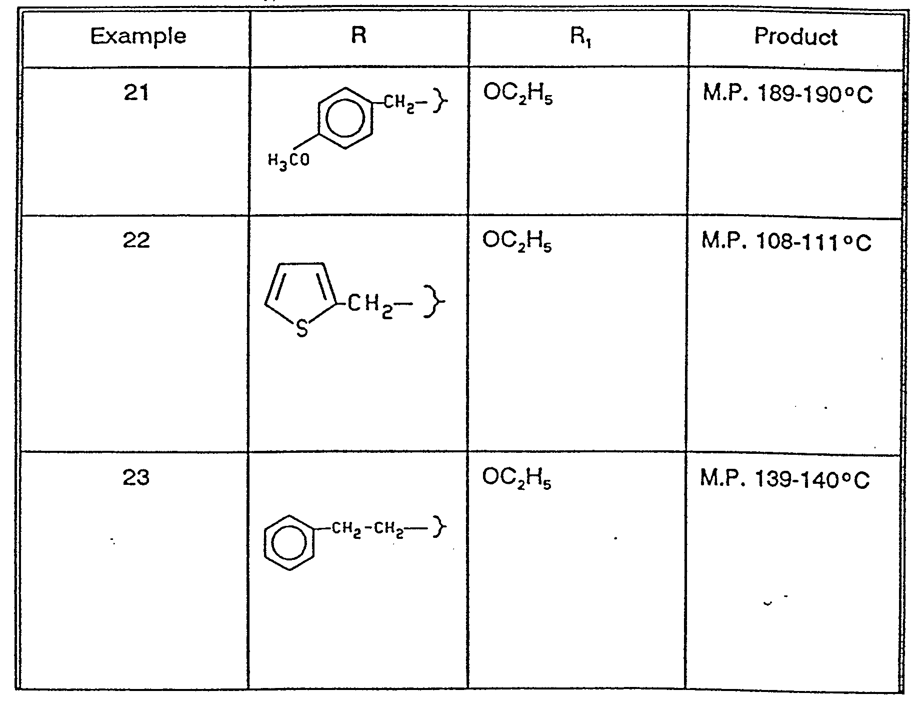

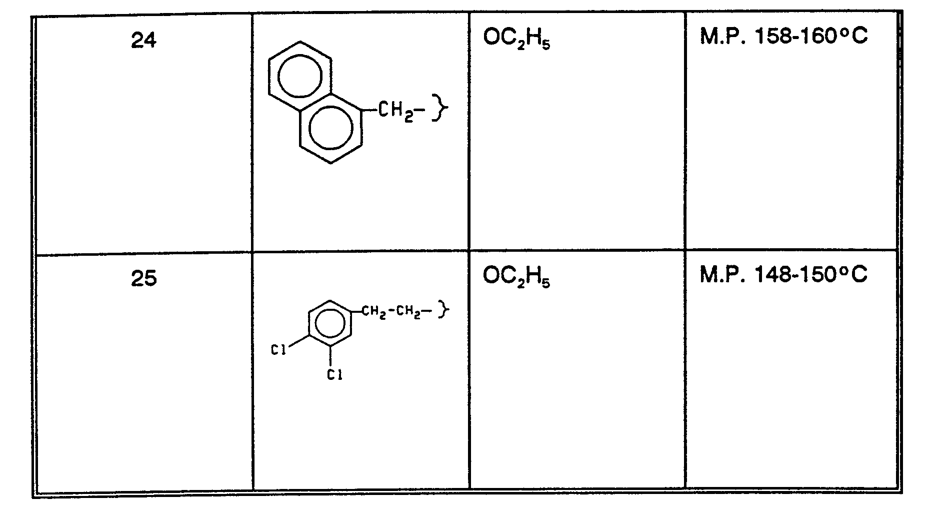

### Example 26

### N-(3-Methylisothiazol-5-yl)-1-(3,4-dichlorobenzyl)-2,4-dioxopyrrolidine-3-carboxamide

A mixture of ethyl 1-(3,4-dichlorobenzyl)-2,4-dioxopyrrolidine-3-carboxylate (0.6 g), 5-amino-3-methylisothiazole (0.26 g) and toluene (50 ml) was refluxed for 15 minutes. The reaction was cooled to room temperature and the precipitated solid was collected (yield: 0.51 g; m.p. > 23°C).

The compounds of Example 27-37 have formula II and were prepared according to the procedure of Example 26.

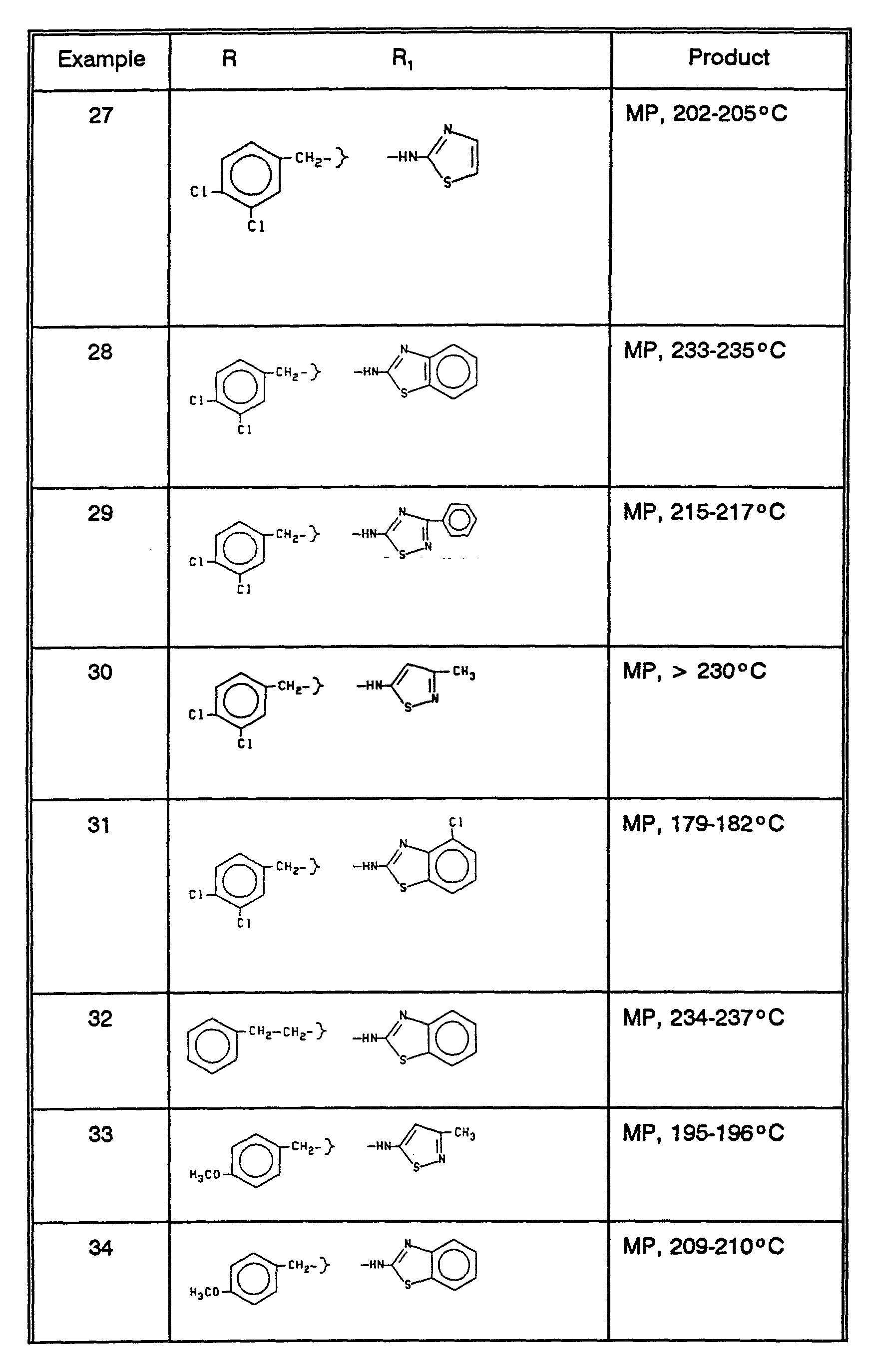

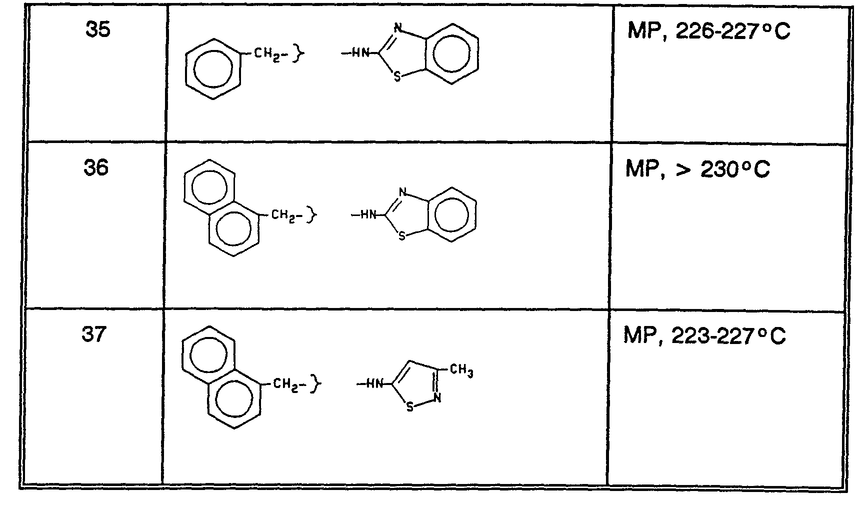

### Example 38

### 2-H-1-Benzopyran-4-carboxylic acid, 6-fluoro-3,4-dihydro-, phenylmethyl ester

A solution of 6-fluoro-3,4-dihydro-2H-1-benzopyran-4-carboxylic acid (50.0 g) in saturated aqueous sodium bicarbonate (200 ml) was added to a solution of benzyl bromide (42.8 g) in methylene chloride (200 ml) containing benzyl tributyl ammonium chloride (78.2 g). The resulting mixture was stirred overnight at room temperature. The reaction mixture was then washed with water (3 x 30 ml), and the methylene chloride layer was collected and evaporated to dryness. The residue was purified by flash chromatography on silica gel using methylene chloride (yield: 74%; ¹H NMR (CDCI₃) δ 2.1 (m, 2H), 3.6 (t, 1H), 4.0 (m, 2H), 5.0 (s, 2H), 6.8 (m, 3H), 7.1 (s, 5H)).

### Example 39

### 2H-1-Benzopyran-4-acetic acid, 6-fluoro-3,4-dihydro-4-[(phenylmethoxy)carbonyl], ethyl ester

To a suspension of sodium hydride (1.05 g, 22 mmol) in dimethylformamide (DMF) (15 ml) was added a solution of 2H-1-benzopyran-4-acetic acid, 6-fluoro-3,4-dihydro-4-[(phenylmethoxy)carbonyl]-, ethyl ester (5.72 g) in DMF (5 ml). After 15 minutes, ethyl bromoacetate (3.34 g) was slowly added. The reaction was complete immediately after the addition. The reaction mixture was poured onto cold water (50 ml). Sufficient hydrochloric acid was added to adjust the pH to about 4.0 and then the mixture was extracted with ethyl acetate (2 x 25 ml). The extract was washed with water, collected, dried and then evaporated to a pale yellow oil (yield: 80% ¹H NMR (CDCI₃) δ 1.2 (t, J=8 Hz, 3H), 2.2 (m, 2H), 2.8-3.2 (m, 2H), 4.1 (m, 4H), 5.1 (s, 2H), 6.9-7.2 (m, 8H)).

### Example 40

### 2H-1-Benzopyran-4-acetic acid-4-carboxylic acid

To a solution of 2H-1-benzopyran-4-acetic acid, 6-fluoro-3,4-dihydro-4-[(phenylmethoxy)carbonyl]-, ethyl ester (5.0 g) in methanol (40 ml) was added a palladium on carbon catalyst (0.5 g) and the mixture was hydrogenated in a Parr apparatus at 50 psi for 1.5 hours. The mixture was filtered and the filtrate was evaporated to obtain the desired compound (yield: 80%; ¹H NMR (CDCI₃) δ 1.2 (t, J=8 Hz, 3H), 2.2 (m, 2H), 2.6-2.9 (m, 2H), 3.9-4.3 (m, 4H), 5.1 (s, 2H), 6.8-7.2 (m, 8H)).

### Example 41

### 2H-1-Benzopyran-4-acetic acid, 6-fluoro-3,4-dihydro-4-[[[(4-methoxyphenyl)methoxy]carbonyl]amino]-, ethyl ester

To a solution of 2H-1-benzopyran-4-acetic acid-4-carboxylic acid (2.82 g) in benzene (75 ml) and triethylamine (1.7 ml) was added diphenylphosphoryl azide (3.30 g). After stirring the reaction mixture overnight, p-methoxybenzyl alcohol (1.66 g) was added to it and then the mixture was refluxed for 4 hours. The reaction mixture was cooled, washed successively with sodium carbonate solution (10%, 15 ml), dilute hydrochloric acid (1N, 5 ml) and water (20 ml). The organic portion was collected, dried and then chromatographed to obtain the title compound (yield: 82%; ¹H NMR (CDCI₃) δ 1.2 (t, J=8 Hz, 3H), 2.2 (m, 2H), 2.6-3.2 (m, 2H), 3.1 (s, 3H), 4.1-4.3 (m, 4H), 4.9 (s, 2H), 5.9 (s, H), 6.6-7.3 (m, 7H)).

### Example 42

### 2H-1-Benzopyran-4-acetic acid, 4-amino-6-fluoro-3,4-dihydro-,ethyl ester

A mixture of 2H-1-benzopyran-4-acetic acid, 6-fluoro-3,4-dihydro-4-[[[4-methoxyphenyl)methoxy]carbonyl]amino]-, ethyl ester (3.3 g, 7.49 mmole), methanol (20 ml) and a palladium on carbon catalyst (0.5 g) was hydrogenated in a Paar apparatus at 20 psi for 1 hour. The catalyst was filtered off and the filtrate was evaporated. The residue was extracted with ethyl acetate and the ethyl aetate layer was extracted with dilute hydrochloric acid. The acidic portion was collected, adjusted to pH 7.0 with 10% potassium hydroxide and extracted with ether. The ether layer was collected, dried and evaporated to obtain the title compound as an oil (14%); ¹H NMR (CDCI₃) δ 1.2 (t, J=8 Hz, 3H), 2.2 (m, 2H), 3.2 (q, J=8 Hz, 2H), 4.1-4.4 (m, 4H), 6.6-7.2 (m, 3H).

### Example 43

### Spiro [4H-1-benzopyran-4,2'-pyrrolidine]3'-carboxylic acid, 6-fluoro-2,3-dihydro-4',5'-dioxo, ethyl ester

To a solution of sodium ethoxide prepared from sodium (11 mg) and ethanol (5 ml) was added a solution of the title compound of Example 12 (75 mg) and diethyl oxalate (58 mg) in ethanol (2 ml). The reaction mixture was stirred overnight, and to the precipitated white solid was added sufficient 10% hydrochloric acid to adjust the pH to about 2.0. The resulting solid was collected and air-dried (yield: 84%; mp 211-214°C; ¹H NMR (CDCI₃) δ 1.05 (t, J=8 Hz, 3H), 2.8 (m, 2H), 4.4 (q, J=8 Hz, 2H), 4.55 (m, 2H), 7.1 (m, 1H), 7.4 (m, 2H)).

## Claims (Claims for the following Contracting State(s): AT, BE, CH, LI, DE, DK, FR, GB, IT, LU, NL, SE)

1. A compound of the formula I 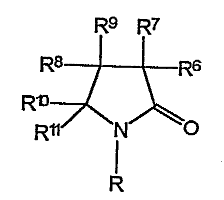 wherein either
(1) R is 9-florenyl or aralkyl wherein the alkyl moiety contains one or two carbon atoms and wherein the aryl moiety is an aromatic or heteroaromatic ring selected from phenyl, furyl, thienyl, pyridyl and naphthyl, and said aromatic or heteroaromatic ring is optionally substituted with one or two substituents independently selected from bromine, chlorine, fluorine, (C₁-C₃) alkyl and (C₁-C₃) alkoxy;
R⁶ and R⁷ together form an oxo group;
R⁸ is hydrogen;
R⁹ is COR¹;
R¹ is OR or NHR³, wherein R is (C₁-C₃) alkyl and R³ is selected from furyl, thienyl, 2-thiazolyl, 2-benzothiazolyl, 3-methyl-5-isothiazolyl and 5-phenyl-1,3,4-thiazolyl;
and R¹⁰ and R¹¹ are each hydrogen; or
(2) R is as defined above;
R⁶ is hydrogen;
R⁷ is CONHR³ wherein R³ is as defined above;
R⁸ and R⁹ together form an oxo group; and
R¹⁰ and R¹¹ are each hydrogen; or
(3) R is hydrogen;
R⁶ and R⁷ together form an oxo group;
R⁸ is hydrogen;
R⁹ is COR¹ wherein R¹ is as defined above;
R¹⁰ and R¹¹ together with the carbon atom to which they are attached form an optionally substituted, spiro-fused bicyclic system of the formula: 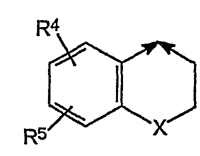
wherein X is methylene, oxygen or sulphur and R⁴ and R⁵ are independently
selected from hydrogen,bromine, chlorine, fluorine and (C-C₃)alkoxy; with the proviso that when R¹ is ethoxy or methoxy, R is other than benzyl or substituted benzyl, wherein the substituents are 2-, 3-, or 4-chloro, 4-methoxy or 3,4-dimethoxy groups; phenethyl or substituted phenethyl wherein the substituents are 4-chloro or 4-methyl;
and the pharmaceutically acceptable salts thereof.

2. A compound according to claim 1, having the formula I wherein the substituent groups R and R¹ are as defined in claim 1

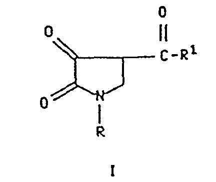

3. A compound according to claim 1, having the formula II wherein the substituent groups R and R³ are as defined in claim 1

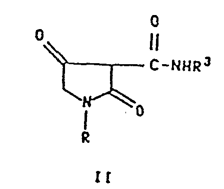

4. A compound according to claim 1, having the formula III wherein the substituent groups R¹, R⁴, R⁵ and X are as defined in claim 1

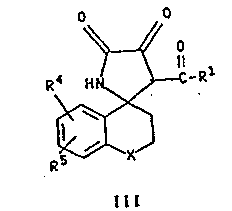

5. A compound according to claim 2, wherein R¹ is ethoxy or propoxy and R is 9-florenyl or aralkyl wherein
the alkyl moiety is methylene and the aryl moiety is selected from phenyl, naphthyl, 2-thienyl, 4-pyridyl, 2-pyridyl, 2-furyl, 4-bromophenyl and 3,4-dichlorophenyl.

6. A compound according to claim 3, wherein R is aralkyl wherein the alkyl moiety is methylene and the aryl moiety is selected from phenyl, 3,4-dichlorophenyl and 4-methoxyphenyl, and R³ is selected from 3-methylisothiazol-3-yl, benzothiazol-2-yl, 5-methylisothiazol-3-yl, thiazol-2-yl and 3-phenyl-1,2,4-thiazol-5-yl.

7. A compound according to claim 4, wherein R⁴ is 6-fluoro, X is oxygen and R¹ is ethoxy.

8. A compound according to claim 1, wherein said compound is selected from:
ethyl 1-(thien-2-ylmethyl)-4,5-dioxopyrrolidine-3-carboxylate;
ethyl 1-(pyrid-2-ylmethyl)-4,5-dioxopyrrolidine-3-carboxylate;
ethyl 1-(pyrid-4-ylmethyl)-4,5-dioxopyrrolidine-3-carboxylate;
ethyl 1-(naphth-1-ylmethyl)-4,5-dioxopyrrolidine-3-carboxylate;
ethyl 1-(4-bromobenzyl)-4,5-dioxopyrrolidine-3-carboxylate;
ethyl 1-(fur-2-ylmethyl)-4,5-dioxopyrrolidine-3-carboxylate;
ethyl 1-(3,4-dichlorobenzyl)-4,5-dioxopyrrolidine-3-carboxylate;
propyl 1-(benzyl)-4,5-dioxopyrrolidine-3-carboxylate;
ethyl 1-(9-florenyl)-4,5-dioxopyrrolidine-3-carboxylate;
N-(3-methylisothiazol-5-yl)-1-benzyl-4,5-dioxopyrrolidine-3-carboxamide;
N-(benzothiazol-2-yl)-1-benzyl-4,5-dioxopyrrolidine-3-carboxamide;
N-(5-methyl-isoxazol-3-yl)-1-benzyl-4,5-dioxopyrrolidine-3-carboxamide;
N-(thiazol-2-yl)-1-(3,4-dichlorobenzyl)-2,4-dioxopyrrolidine-3-carboxamide;
N-(benzothiazol-2-yl)-1-(3,4-dichlorobenzyl)-2,4-dioxopyrrolidine-3-carboxamide;
N-(3-phenyl-1,2,4-thiadiazol-5-yl)-1-(3,4-dichlorobenzyl)-2,4-dioxopyrrolidine-3-carboxamide;
N-(benzothiazol-2-yl)-1 -(2-phenylethyl)-2,4-dioxopyrrolidine-3-carboxamide;
N-(3-methyl-isothiazol-5-yl)-1-(4-methoxybenzyl)-2,4-dioxopyrrolidine-3-carboxamide;
N-(benzothiazol-2-yl)-1-(4-methoxybenzyl)-2,4-dioxopyrrolidine-3-carboxamide; and
spiro [4H-1-benzopyran-4,2'-pyrrolidine]3'-carboxylic acid, 6-fluoro-2,3-dihydro-4',5'-dioxo-, ethyl ester; and pharmaceutically acceptable salts thereof.

9. A pharmaceutical composition for preventing or alleviating a diabetes associated complication such as neuropathy, nephropathy, retinopathy and cataracts in a diabetic mammal, said composition comprising a sufficient amount of a compound according to claim 1 effective in preventing or alleviating said complication and a pharmaceutically acceptable carrier.

10. A process for preparing a compound of the formula 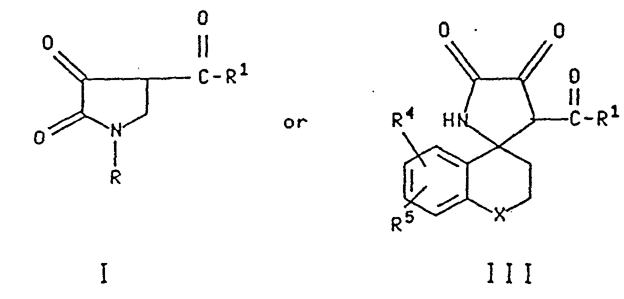 wherein R, R¹, R⁴, R⁵ and X are as defined in claim 1.
or a pharmaceutically acceptable salt thereof; comprising reacting a compound of the formula 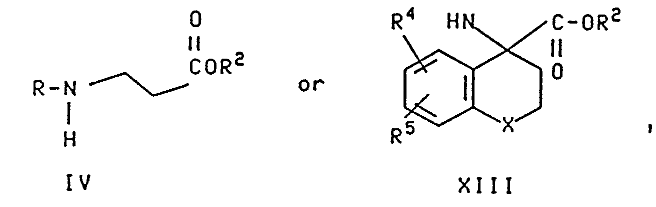 respectively with a compound of the formula 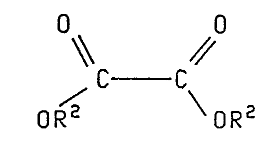 wherein R, R, R⁴, R⁵ and X are as defined above, and if desired preparing a compound of the formula I or III wherein R¹ is R³NH by reacting a compound of the formula I or III, respectively, wherein R¹ is OR with a compound of the formula R³NH₂ wherein R³ is selected from furyl, thienyl, 2-thiazolyl, 2-benzothiazolyl, 3-methyl-5-iothiazolyl and 5-phenyl-1,3,4-thiazolyl; and optionally converting the compounds of formula I or III to pharmaceutically acceptable salts.

11. A process for preparing a compound of the formula 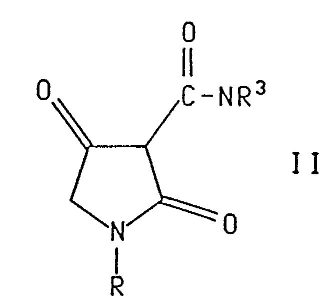 wherein R and R³ are defined as in claim 1 comprising a) treating a compound of the formula 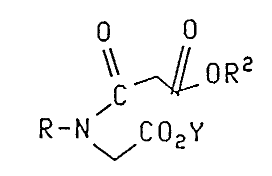 wherein R and R are defined as in claim 1 and Y is (C₁-C₃) alkyl with a suitable alkali or alkaline earth alkoxide and b) reacting the product of step a) with a compound of the formula R³NH₂ wherein R³ is as defined above; and optionally converting the product of said reaction to a pharmaceutically acceptable salt.

12. A process according to claim 10 for preparing a compound of formula I wherein R¹ is ethoxy or propoxy and R is selected from 9-florenyl or aralkyl wherein the alkyl moiety is methylene and the aryl moiety is selected from phenyl, naphthyl, 2-thienyl, 4-pyridyl, 2-pyridyl, 2-furyl, and 4-bromophenyl.

13. A process according to claim 10 for preparing a compound of formula III wherein R¹ is ethoxy, R⁴ is 6-fluoro, R⁵ is hydrogen and X is oxygen.

14. A process according to claim 10 for preparing a compound of formula I wherein R¹ is NHR³ and R, R³ are defined as in claim 1.

15. A process according to claim 10 for preparing a compound of formula II wherein R is aralkyl wherein the alkyl moiety is methylene and the aryl moiety is selected from phenyl, 3,4-dichlorophenyl and 4-methoxyphenyl and R³ is selected from 3-methylisothiazol-3-yl, benzothiazole-2-yl, 5-methylisothiazol-3-yl, thiazol-2-yl and 3-phenyl-1,2,4-thiazol-5-yl.

16. A process according to claim 10, for preparing a compound of the formula I selected from
ethyl 1-(thien-2-ylmethyl)-4,5-dioxopyrrolidine-3-carboxylate;
ethyl 1-(pyrid-2-ylmethyl)-4,5-dioxopyrrolidine-3-carboxylate;
ethyl 1-(pyrid-4-ylmethyl)-4,5-dioxopyrrolidine-3-carboxylate;
ethyl 1-(naphth-1-ylmethyl)-4,5-dioxopyrrolidine-3-carboxylate;
ethyl 1-(4-bromobenzyl)-4,5-dioxopyrrolidine-3-carboxylate;
ethyl 1-(fur-2-ylmethyl)-4,5-dioxopyrrolidine-3-carboxylate;
ethyl 1-(3,4-dichlorobenzyl)-4,5-dioxopyrrolidine-3-carboxylate;
propyl 1-(benzyl)-4,5-dioxopyrrolidine-3-carboxylate;
ethyl 1-(9-florenyl)-4,5-dioxopyrrolidine-3-carboxylate and pharmaceutically acceptable salts thereof.

17. A process according to claim 10 for preparing a compound of formula I selected from
N-(3-methylisothiazol-5-yl)-1-benzyl-4,5-dioxopyrrolidine-3-carboxamide;
N-(benzothiazol-2-yl)-1-benzyl-4,5-dioxopyrrolidine-3-carboxamide;
N-(5-methyl-isoxazol-3-yl)-1-benzyl-4,5-dioxopyrrolidine-3-carboxamide; and pharmaceutically accepable salts thereof.

18. A process according to claim 10 for preparing spiro [4H-1-benzopyran-4,2'-pyrrolidine]3'-carboxylic acid, 6-fluoro-2,3-dihydro-4',5'-dioxo-, ethyl ester and pharmaceutically acceptable salts thereof.

19. A process according to claim 11 for preparing a compound of formula II selected from
N-(thiazol-2-yl)-1-(3,4-dichlorobenzyl)-2,4-dioxopyrrolidine-3-carboxamide;
N-(3-phenyl-1,2,4-thiadiazol-5-yl)-1-(3,4-dichlorbenzyl)-2,4-dioxopyrrolidine-3-carboxamide;
N-(benzothiazol-2-yl)-1-(2-phenethyl)-2,4-dioxopyrrolidine-3-carboxamide;
N-(3-methyl-isothiazol-5-yl)-1-(4-methoxybenzyl)-2,4-dioxopyrrolidine-3-carboxamide;
N-(benzothiazol-2-yl)-1-(4-methoxybenzyl)-2,4-dioxopyrrolidine-3-carboxamide; and pharmaceutically acceptable salts thereof.

## Claims (Claims for the following Contracting State(s): ES, GR)

1. A process for preparing a compound of the formula 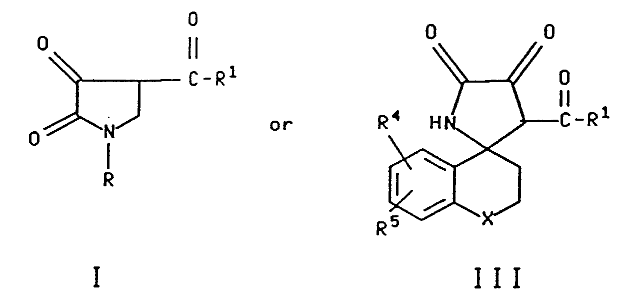 wherein
R is 9-florenyl or aralkyl wherein the alkyl moiety contains one or two carbon atoms and wherein the aryl moiety is an aromatic or heteroaromatic ring selected from phenyl, furyl, thienyl, pyridyl and naphthyl, said aromatic or heteroaromatic ring being optionally substituted with one or two substituents independently selected from bromine, chlorine, fluorine, (C₁-C₃) alkyl and (C₁-C₃) alkoxy;
R¹ is OR; wherein R is (C₁-C₃) alkyl;
R⁴ and R⁵ are independently selected from hydrogen, bromine, chlorine, fluorine and (C₁-C₃) alkoxy; and
X is methylene, oxygen or sulfur;
with the proviso that when R¹ is ethoxy or methoxy, R is other than benzyl or substituted benzyl, wherein the substituents are 2-, 3-, or 4-chloro, 4-methoxy or 3,4-dimethoxy groups; phenethyl or substituted phenethyl wherein the substituents are 4-chloro or 4-methyl;
or a pharmaceutically acceptable salt thereof; comprising reacting a compound of the formula 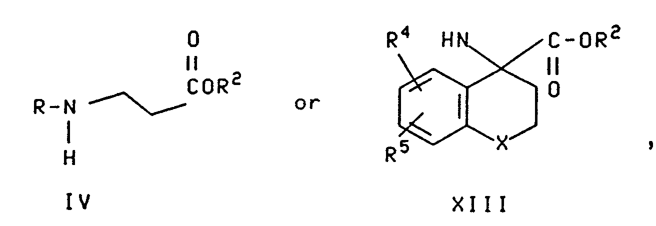 respectively with a compound of the formula 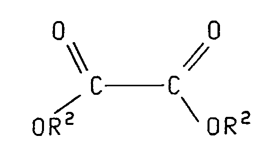 wherein R, R, R⁴, R⁵ and X are as defined above, and if desired preparing a compound of the formula I or III wherein R¹ is R³NH by reacting a compound of the formula I or III, respectively, wherein R¹ is OR with a compound of the formula R³NH₂ wherein R³ is selected from furyl, thienyl, 2-thiazolyl, 2-benzothiazolyl, 3-methyl-5-isothiazolyl and 5-phenyl-1,3,4-thiazolyl; and optionally converting the compounds of formula I or III to pharmaceutically acceptable salts.

2. A process for preparing a compound of the formula 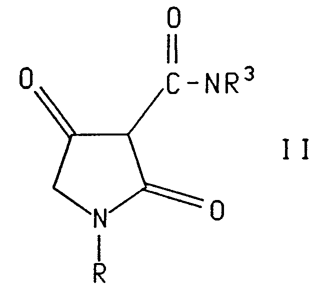 wherein R and R³ are defined as in claim 1 comprising a) treating a compound of the formula 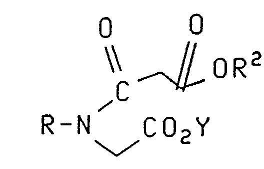 wherein R and R are defined as in claim 1 and Y is (C₁-C₃) alkyl with a suitable alkali or alkaline earth alkoxide and b) reacting the product of step a) with a compound of the formula R³NH₂ wherein R³ is as defined above; and optionally converting the product of said reaction to a pharmaceutically acceptable salt.

3. A process according to claim 1 for preparing a compound of formula I wherein R¹ is ethoxy or propoxy and R is selected from 9-florenyl or aralkyl wherein the alkyl moiety is methylene and the aryl moiety is selected from phenyl, naphthyl, 2-thienyl, 4-pyridyl, 2-pyridyl, 2-furyl, and 4-bromophenyl.

4. A process according to claim 1 for preparing a compound of formula III wherein R¹ is ethoxy, R⁴ is 6-fluoro, R⁵ is hydrogen and X is oxygen.

5. A process according to claim 1 for preparing a compound of formula I wherein R¹ is NHR³ and R, R³ are defined as in claim 1.

6. A process according to claim 1 for preparing a compound of formula II wherein R is aralkyl wherein the alkyl moiety is methylene and the aryl moiety is selected from phenyl, 3,4-dichlorophenyl and 4-methoxyphenyl and R³ is selected from 3-methylisothiazol-3-yl, benzothiazole-2-yl, 5-methylisothiazol-3-yl, thiazol-2-yl and 3-phenyl-1,2,4-thiazol-5-yl.

7. A process according to claim 1, for preparing a compound of the formula I selected from
ethyl 1-(thien-2-ylmethyl)-4,5-dioxopyrrolidine-3-carboxylate;
ethyl 1-(pyrid-2-ylmethyl)-4,5-dioxopyrrolidine-3-carboxylate;
ethyl 1-(pyrid-4-ylmethyl)-4,5-dioxopyrrolidine-3-carboxylate;
ethyl 1-(naphth-1-ylmethyl)-4,5-dioxopyrrolidine-3-carboxylate;
ethyl 1-(4-bromobenzyl)-4,5-dioxopyrrolidine-3-carboxylate;
ethyl 1-(fur-2-ylmethyl)-4,5-dioxopyrrolidine-3-carboxylate;
ethyl 1-(3,4-dichlorobenzyl)-4,5-dioxopyrrolidine-3-carboxylate;
propyl 1-(benzyl)-4,5-dioxopyrrolidine-3-carboxylate;
ethyl 1-(9-florenyl)-4,5-dioxopyrrolidine-3-carboxylate and pharmaceutically acceptable salts thereof.

8. A process according to claim 5 for preparing a compound of formula I selected from
N-(3-methylisothiazol-5-yl)-1-benzyl-4,5-dioxopyrrolidine-3-carboxamide;
N-(benzothiazol-2-yl)-1-benzyl-4,5-dioxopyrrolidine-3-carboxamide;
N-(5-methyl-isoxazol-3-yl)-1-benzyl-4,5-dioxopyrrolidine-3-carboxamide; and pharmaceutically accepable salts thereof.

9. A process according to claim 4 for preparing spiro [4H-1-benzopyran-4,2'-pyrrolidine]3'-carboxylic acid, 6-fluoro-2,3-dihydro-4',5'-dioxo-, ethyl ester and pharmaceutically acceptable salts thereof.

10. A process according to claim 6 for preparing a compound of formula II selected from
N-(thiazol-2-yl)-1-(3,4-dichlorobenzyl)-2,4-dioxopyrrolidine-3-carboxamide;
N-(3-phenyl-1,2,4-thiadiazol-5-yl)-1-(3,4-dichlorbenzyl)-2,4-dioxopyrrolidine-3-carboxamide;
N-(benzothiazol-2-yl)-1-(2-phenethyl)-2,4-dioxopyrrolidine-3-carboxamide;
N-(3-methyl-isothiazol-5-yl)-1-(4-methoxybenzyl)-2,4-dioxopyrrolidine-3-carboxamide;
N-(benzothiazol-2-yl)-1-(4-methoxybenzyl)-2,4-dioxopyrrolidine-3-carboxamide; and pharmaceutically acceptable salts thereof.

## Patentansprüche (Patentansprüche für folgende(n) Vertragsstaat(en): AT, BE, CH, LI, DE, DK, FR, GB, IT, LU, NL, SE)

1. Verbindung der Formel 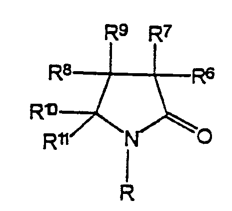 worin entweder
(1) R 9-Florenyl oder Aralkyl darstellt, worin der Alkylrest ein oder zwei Kohlenstoffatome enthält und worin der Arylrest einen aromatischen oder heteroaromatischen Ring darstellt, ausgewählt aus Phenyl, Furyl, Thienyl, Pyridyl und Naphthyl und der aromatische oder heteroaromatische Ring gegebenenfalls substituiert ist mit einem oder zwei Substituenten, unabhängig ausgewählt aus Brom, Chlor, Fluor, (C₁-C₃)-Alkyl und (C₁-C₃)-Alkoxy;
R⁶ und R⁷ zusammen eine Oxogruppe bilden;
R⁸ Wasserstoff darstellt;
R⁹ COR¹ darstellt;
R¹ OR oder NHR³ darstellt, worin R (C₁-C₃)-Alkyl darstellt und R³ ausgewählt ist aus Furyl, Thienyl, 2-Thiazolyl, 2-Benzothiazolyl, 3-Methyl-5-isothiazolyl und 5-Phenyl-1,3,4-thiazolyl;
und R¹⁰ und R¹¹ jeweils Wasserstoff darstellen; oder
(2) R wie vorstehend definiert ist;
R⁶ Wasserstoff darstellt;
R⁷ CONHR³ darstellt, worin R³ wie vorstehend definiert ist;
R⁸ und R⁹ zusammen eine Oxogruppe bilden; und
R¹⁰ und R¹¹ jeweils Wasserstoff darstellen; oder
(3) R Wasserstoff darstellt;
R⁶ und R⁷ zusammen eine Oxogruppe bilden;
R⁸ Wasserstoff darstellt;
R⁹ COR¹ darstellt, worin R¹ wie vorstehend definiert ist;
R¹⁰ und R¹¹ zusammen mit dem Kohlenstoffatom, an das sie gebunden sind, ein gegebenenfalls substituiertes spirokondensiertes, bicyclisches System bilden der Formel: 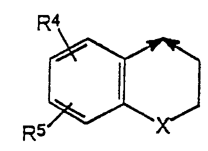
worin X Methylen, Sauerstoff oder Schwefel darstellt und R⁴ und R⁵ unabhängig ausgewählt sind aus Wasserstoff, Brom, Chlor, Fluor und (C-C₃)-Alkoxy;
mit der Maßgabe, daß wenn R¹ Ethoxy oder Methoxy ist, R nicht Benzyl oder substituiertes Benzyl, worin die Substituenten 2-, 3- oder 4-Chlor, 4-Methoxy oder 3,4-Dimethoxy-gruppen sind; Phenethyl oder substituiertes Phenethyl darstellt, worin die Substituenten 4-Chlor oder 4-Methyl sind;
und die pharmazeutisch verträglichen Salze davon.

2. Verbindung nach Anspruch 1 mit der Formel I, worin die Substituentengruppen R und R¹ wie in Anspruch 1 definiert sind

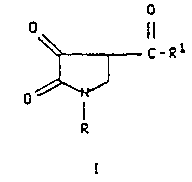

3. Verbindung nach Anspruch 1 mit der Formel II, worin die Substituentengruppen R und R³ wie in Anspruch 1 definiert sind

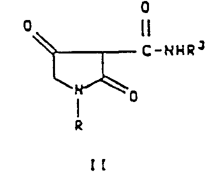

4. Verbindung nach Anspruch 1 mit der Formel III, worin die Substituentengruppen R¹, R⁴, R⁵ und X wie in Anspruch 1 definiert sind

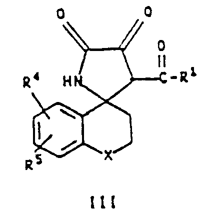

5. Verbindung nach Anspruch 2, worin R¹ Ethoxy oder Propoxy darstellt und R 9-Florenyl oder Aralkyl darstellt, worin
der Alkylrest Methylen ist und der Arylrest ausgewählt ist aus Phenyl, Naphthyl, 2-Thienyl, 4-Pyridyl, 2-Pyridyl, 2-Furyl, 4-Bromphenyl und 3,4-Dichlorphenyl.

6. Verbindung nach Anspruch 3, worin R Aralkyl darstellt, worin der Alkylrest Methylen ist und der Arylrest ausgewählt ist aus Phenyl, 3,4-Dichlorphenyl und 4-Methoxyphenyl und R³ ist ausgewählt aus 3-Methylisothiazol-3-yl, Benzothiazol-2-yl, 5-Methylisothiazol-3-yl, Thiazol-2-yl und 3-Phenyl-1,2,4-thiazol-5-yl.

7. Verbindung nach Anspruch 4, worin R⁴ 6-Fluor darstellt, X Sauerstoff darstellt und R¹ Ethoxy darstellt.

8. Verbindung nach Anspruch 1, worin die Verbindung ausgewählt ist aus:
1-(Thien-2-ylmethyl)-4,5-dioxopyrrolidin-3-carbonsäureethylester;
1-(Pyrid-2-ylmethyl)-4,5-dioxopyrrolidin-3-carbonsäureethylester;
1-(Pyrid-4-ylmethyl)-4,5-dioxopyrrolidin-3-carbonsäureethylester;
1-(Naphth-1-ylmethyl)-4,5-dioxopyrrolidin-3-carbonsäureethylester;
1-(4-Brombenzyl)-4,5-dioxopyrrolidin-3-carbonsäure-ethylester;
1-(Fur-2-ylmethyl)-4,5-dioxopyrrolidin-3-carbonsäure-ethylester;
1-(3,4-Dichlorbenzyl)-4,5-dioxopyrrolidin-3-carbonsäure-ethylester;
1-(Benzyl)-4,5-dioxopyrrolidin-3-carbonsäurepropylester;
1-(9-Florenyl)-4,5-dioxopyrrolidin-3-carbonsäureethylester;
N-(3-Methylisothiazol-5-yl)-1-benzyl-4,5-dioxopyrrolidin-3-carboxamid;
N-(Benzothiazol-2-yl)-1-benzyl-4,5-dioxopyrrolidin-3-carboxamid;
N-(5-Methylisoxazol-3-yl)-1-benzyl-4,5-dioxopyrrolidin-3-carboxamid;
N-(Thiazol-2-yl)-1-(3,4-dichlorbenzyl)-2,4-dioxopyrrolidin-3-carboxamid;
N-(Benzothiazol-2-yl)-1-(3,4-dichlorbenzyl)-2,4-dioxopyrrolidin-3-carboxamid;
N-(3-Phenyl-1,2,4-thiadiazol-5-yl)-1-(3,4-dichlorbenzyl)-2,4-dioxopyrrolidin-3-carboxamid;
N-(Benzothiazol-2-yl)-1-(2-phenylethyl)-2,4-dioxopyrrolidin-3-carboxamid;
N-(3-Methylisothiazol-5-yl)-1-(4-methoxybenzyl)-2,4-dioxopyrrolidin-3-carboxamid;
N-(Benzothiazol-2-yl)-1-(4-methoxybenzyl)-2,4-dioxopyrrolidin-3-carboxamid; und
Spiro-[4H-1-benzopyran-4,2'-pyrrolidin]-3'-carbonsäure-6-fluor-2,3-dihydro-4',5'-dioxo-ethylester;
und pharmazeutisch verträgliche Salze davon.

9. Pharmazeutische Zusammensetzung zur Verhinderung oder Linderung einer mit Diabetes verbundenen Komplikation, wie Nervenleiden, Nierenleiden, Netzhauterkrankung und Linsentrübung, bei einem diabetischen Säuger, wobei die Zusammensetzung eine ausreichende Menge einer Verbindung nach Anspruch 1, wirksam zur Verhinderung oder Linderung der Komplikation und einen pharmazeutisch verträglichen Träger umfaßt.

10. Verfahren zur Herstellung einer Verbindung der Formel 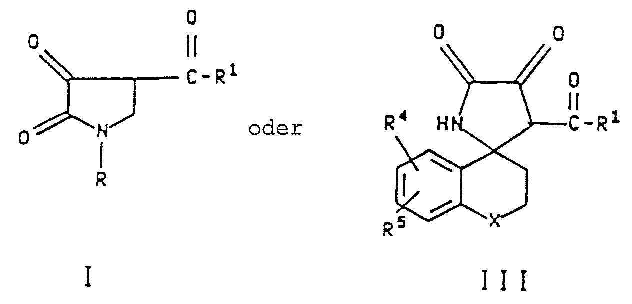
worin R, R¹, R⁴, R⁵ und X wie in Anspruch 1 definiert sind
oder eines pharmazeutisch verträglichen Salzes davon; umfassend Umsetzung einer Verbindung der Formel 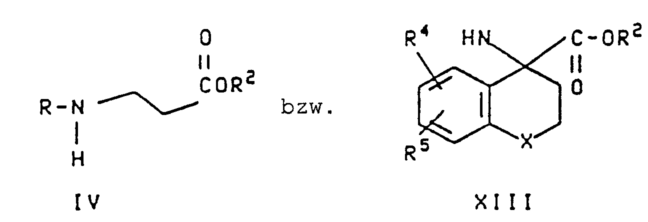
mit einer Verbindung der Formel 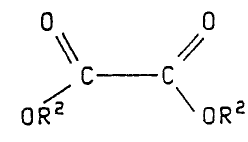
worin R, R, R⁴, R⁵ und X wie vorstehend definiert sind und falls erwünscht, Herstellen einer Verbindung der Formel I oder III, worin R¹ R³NH darstellt, durch Umsetzen einer Verbindung der Formel I bzw. III, worin R¹ OR darstellt mit einer Verbindung der Formel R³NH₂, worin R³ ausgewählt ist aus Furyl, Thienyl, 2-Thiazolyl, 2-Benzothiazolyl, 3-Methyl-5-isothiazolyl und 5-Phenyl-1,3,4-thiazolyl; und gegebenenfalls Umwandeln der Verbindungen der Formeln I oder III in pharmazeutisch verträgliche Salze.

11. Verfahren zur Herstellung einer Verbindung der Formel 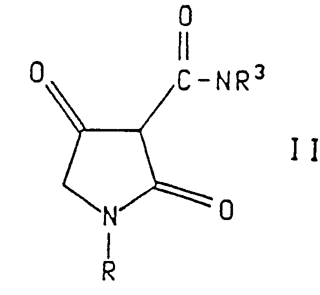
worin R und R³ wie in Anspruch 1 definiert sind, umfassend a) Behandeln einer Verbindung der Formel 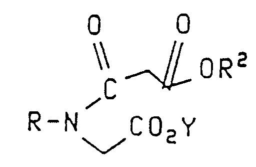
worin R und R wie in Anspruch 1 definiert sind und Y (C₁-C₃)-Alkyl darstellt, mit einem geeigneten Alkali- oder Erdalkalialkoxid und b) Umsetzen des Produkts von Schritt a) mit einer Verbindung der Formel R³NH₂, worin R³ wie vorstehend definiert ist; und gegebenenfalls Umwandeln des Produkts der Reaktion in ein pharmazeutisch verträgliches Salz.

12. Verfahren nach Anspruch 10 zur Herstellung einer Verbindung der Formel I, worin R¹ Ethoxy oder Propoxy darstellt und R ausgewählt ist aus 9-Florenyl oder Aralkyl, worin der Alkylrest Methylen darstellt und der Arylrest ausgewählt ist aus Phenyl, Naphthyl, 2-Thienyl, 4-Pyridyl, 2-Pyridyl, 2-Furyl und 4-Bromphenyl.

13. Verfahren nach Anspruch 10 zur Herstellung einer Verbindung der Formel III, worin R¹ Ethoxy darstellt, R⁴ 6-Fluor darstellt, R⁵ Wasserstoff darstellt und X Sauerstoff darstellt.

14. Verfahren nach Anspruch 10 zur Herstellung einer Verbindung der Formel I, worin R¹ NHR³ darstellt und R, R³ wie in Anspruch 1 definiert sind.

15. Verfahren nach Anspruch 10 zur Herstellung einer Verbindung der Formel II, worin R Aralkyl ist, worin der Alkylrest Methylen darstellt und der Arylrest ausgewählt ist aus Phenyl, 3,4-Dichlorphenyl und 4-Methoxyphenyl und R³ ausgewählt ist aus 3-Methylisothiazol-3-yl, Benzothiazol-2-yl, 5-Methylisothiazol-3-yl, Thiazol-2-yl und 3-Phenyl-1,2,4-thiazol-5-yl.

16. Verfahren nach Anspruch 10 zur Herstellung einer Verbindung der Formel I, ausgewählt aus
1-(Thien-2-ylmethyl)-4,5-dioxopyrrolidin-3-carbonsäureethylester;
1-(Pyrid-2-ylmethyl)-4,5-dioxopyrrolidin-3-carbonsäureethylester;
1-(Pyrid-4-ylmethyl)-4,5-dioxopyrrolidin-3-carbonsäureethylester;
1-(Naphth-1-ylmethyl)-4,5-dioxopyrrolidin-3-carbonsäureethylester;
1-(4-Brombenzyl)-4,5-dioxopyrrolidin-3-carbonsäureethylester;
1-(Fur-2-ylmethyl)-4,5-dioxopyrrolidin-3-carbonsäureethylester;
1-(3,4-Dichlorbenzyl)-4,5-dioxopyrrolidin-3-carbonsäureethylester;
1-(Benzyl)-4,5-dioxopyrrolidin-3-carbonsäurepropylester;
1-(9-Florenyl)-4,5-dioxopyrrolidin-3-carbonsäureethylester;
und pharmazeutisch verträglichen Salzen davon.

17. Verfahren nach Anspruch 10 zur Herstellung einer Verbindung der Formel I, ausgewählt aus
N-(3-Methylisothiazol-5-yl)-1-benzyl-4,5-dioxopyrrolidin-3-carboxamid;
N-(Benzothiazol-2-yl)-1-benzyl-4,5-dioxopyrrolidin-3-carboxamid;
N-(5-Methylisoxazol-3-yl)-1-benzyl-4,5-dioxopyrrolidin-3-carboxamid; und pharmazeutisch verträglichen Salzen davon.

18. Verfahren nach Anspruch 10 zur Herstellung von
Spiro-[4H-1-benzopyran-4,2'-pyrrolidin]-3'-carbonsäure-6-fluor-2,3-dihydro-4',5'-dioxo-ethylester;
und pharmazeutisch verträglichen Salzen davon.

19. Verfahren nach Anspruch 11 zur Herstellung einer Verbindung der Formel II, ausgewählt aus
N-(Thiazol-2-yl)-1-(3,4-dichlorbenzyl)-2,4-dioxopyrrolidin-3-carboxamid;
N-(3-Phenyl-1,2,4-thiadiazol-5-yl)-1-(3,4-dichlorbenzyl)-2,4-dioxopyrrolidin-3-carboxamid;
N-(Benzothiazol-2-yl)-1-(2-phenylethyl)-2,4-dioxopyrrolidin-3-carboxamid;
N-(3-Methylisothiazol-5-yl)-1-(4-methoxybenzyl)-2,4-dioxopyrrolidin-3-carboxamid;
N-(Benzothiazol-2-yl)-1-(4-methoxybenzyl)-2,4-dioxopyrrolidin-3-carboxamid; und pharmazeutisch verträglichen Salzen davon.

## Patentansprüche (Patentansprüche für folgende(n) Vertragsstaat(en): ES, GR)

1. Verfahren zur Herstellung einer Verbindung der Formel 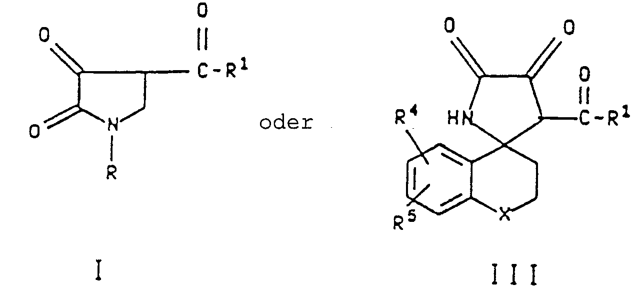 worin
R 9-Florenyl oder Aralkyl darstellt, worin der Alkylrest ein oder zwei Kohlenstoffatome enthält und worin der Arylrest einen aromatischen oder heteroaromatischen Ring darstellt, ausgewählt aus Phenyl, Furyl, Thienyl, Pyridyl und Naphthyl und der aromatische oder heteroaromatische Ring gegebenenfalls substituiert ist mit einem oder zwei Substituenten, unabhängig ausgewählt aus Brom, Chlor, Fluor, (C₁-C₃)-Alkyl und (C₁-C₃)-Alkoxy;
R¹ OR darstellt, worin R (C₁-C₃)-Alkyl darstellt;
R⁴ und R⁵ unabhängig ausgewählt sind aus Wasserstoff, Brom, Chlor, Fluor und (C₁-C₃)-Alkoxy; und
X Methylen, Sauerstoff oder Schwefel darstellt;
mit der Maßgabe, daß wenn R¹ Ethoxy oder Methoxy ist, R nicht Benzyl oder substituiertes Benzyl, worin die Substituenten 2-, 3- oder 4-chlor, 4-Methoxy oder 3,4-Dimethoxygruppen sind; Phenethyl oder substituiertes Phenethyl darstellt, worin die Substituenten 4-Chlor oder 4-Methyl sind;
oder eines pharmazeutisch verträglichen Salzes davon; umfassend Umsetzung einer Verbindung der Formel 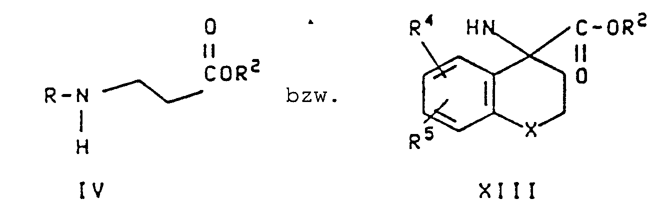
mit einer Verbindung der Formel 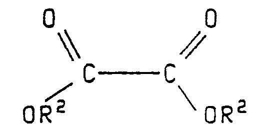
worin R, R, R⁴, R⁵ und X wie vorstehend definiert sind und falls erwünscht, Herstellen einer Verbindung der Formel I oder III, worin R¹ R³NH darstellt, durch Umsetzen einer Verbindung der Formel I bzw. III, worin R¹ OR darstellt mit einer Verbindung der Formel R³NH₂, worin R³ ausgewählt ist aus Furyl, Thienyl, 2-Thiazolyl, 2-Benzothiazolyl, 3-Methyl-5-isothiazolyl und 5-Phenyl-1,3,4-thiazolyl; und gegebenenfalls Umwandeln der Verbindungen der Formeln I oder III in pharmazeutisch verträgliche Salze.

2. Verfahren zur Herstellung einer Verbindung der Formel 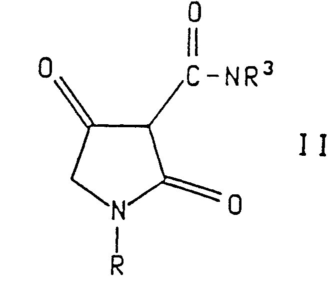
worin R und R³ wie in Anspruch 1 definiert sind, umfassend a) Behandeln einer Verbindung der Formel 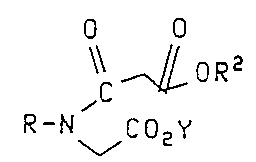
worin R und R wie in Anspruch 1 definiert sind und Y (C₁-C₃)-Alkyl darstellt, mit einem geeigneten Alkali- oder Erdalkalialkoxid und b) Umsetzen des Produkts von Schritt a) mit einer Verbindung der Formel R³NH₂, worin R³ wie vorstehend definiert ist; und gegebenenfalls Umwandeln des Produkts der Reaktion in ein pharmazeutisch verträgliches Salz.

3. Verfahren nach Anspruch 1 zur Herstellung einer Verbindung der Formel I, worin R¹ Ethoxy oder Propoxy darstellt und R ausgewählt ist aus 9-Florenyl oder Aralkyl, worin der Alkylrest Methylen darstellt und der Arylrest ausgewählt ist aus Phenyl, Naphthyl, 2-Thienyl, 4-Pyridyl, 2-Pyridyl, 2-Furyl und 4-Bromphenyl.

4. Verfahren nach Anspruch 1 zur Herstellung einer Verbindung der Formel III, worin R¹ Ethoxy darstellt, R⁴ 6-Fluor darstellt, R⁵ Wasserstoff darstellt und X Sauerstoff darstellt.

5. Verfahren nach Anspruch 1 zur Herstellung einer Verbindung der Formel I, worin R¹ NHR³ darstellt und R, R³ wie in Anspruch 1 definiert sind.

6. Verfahren nach Anspruch 1 zur Herstellung einer Verbindung der Formel II, worin R Aralkyl ist, worin der Alkylrest Methylen darstellt und der Arylrest ausgewählt ist aus Phenyl, 3,4-Dichlorphenyl und 4-Methoxyphenyl und R³ ausgewählt ist aus 3-Methylisothiazol-3-yl, Benzothiazol-2-yl, 5-Methylisothiazol-3-yl, Thiazol-2-yl und 3-Phenyl-1,2,4-thiazol-5-yl.

7. Verfahren nach Anspruch 1 zur Herstellung einer Verbindung der Formel I, ausgewählt aus
1-(Thien-2-ylmethyl)-4,5-dioxopyrrolidin-3-carbonsäureethylester;
1-(Pyrid-2-ylmethyl)-4,5-dioxopyrrolidin-3-carbonsäureethylester;
1-(Pyrid-4-ylmethyl)-4,5-dioxopyrrolidin-3-carbonsäureethylester;
1-(Naphth-1-ylmethyl)-4,5-dioxopyrrolidin-3-carbonsäureethylester;
1-(4-Brombenzyl)-4,5-dioxopyrrolidin-3-carbonsäureethylester;
1-(Fur-2-ylmethyl)-4,5-dioxopyrrolidin-3-carbonsäureethylester;
1-(3,4-Dichlorbenzyl)-4,5-dioxopyrrolidin-3-carbonsäureethylester;
1-(Benzyl)-4,5-dioxopyrrolidin-3-carbonsäurepropylester;
1-(9-Florenyl)-4,5-dioxopyrrolidin-3-carbonsäureethylester;
und pharmazeutisch verträglichen Salzen davon.

8. Verfahren nach Anspruch 5 zur Herstellung einer Verbindung der Formel I, ausgewählt aus
N-(3-Methylisothiazol-5-yl)-1-benzyl-4,5-dioxopyrrolidin-3-carboxamid;
N-(Benzothiazol-2-yl)-1-benzyl-4,5-dioxopyrrolidin-3-carboxamid;
N-(5-Methylisoxazol-3-yl)-1-benzyl-4,5-dioxopyrrolidin-3-carboxamid; und pharmazeutisch verträglichen Salzen davon.

9. Verfahren nach Anspruch 4 zur Herstellung von
Spiro-[4H-1-benzopyran-4,2'-pyrrolidin]-3'-carbonsäure-6-fluor-2,3-dihydro-4',5'-dioxo-ethylester;
und pharmazeutisch verträglichen Salzen davon.

10. Verfahren nach Anspruch 6 zur Herstellung einer Verbindung der Formel II, ausgewählt aus
N-(Thiazol-2-yl)-1-(3,4-dichlorbenzyl)-2,4-dioxopyrrolidin-3-carboxamid;
N-(3-Phenyl-1,2,4-thiadiazol-5-yl)-1-(3,4-dichlorbenzyl)-2,4-dioxopyrrolidin-3-carboxamid;
N-(Benzothiazol-2-yl)-1-(2-phenylethyl)-2,4-dioxopyrrolidin-3-carboxamid;
N-(3-Methylisothiazol-5-yl)-1-(4-methoxybenzyl)-2,4-dioxopyrrolidin-3-carboxamid;
N-(Benzothiazol-2-yl)-1-(4-methoxybenzyl)-2,4-dioxopyrrolidin-3-carboxamid; und pharmazeutisch verträglichen Salzen davon.

## Revendications (Revendications pour l'(les) Etat(s) contractant(s) suivant(s): AT, BE, CH, DE, DK, FR, GB, IT, LI, LU, NL, SE)

1. Composé de formule 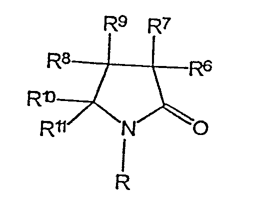 dans laquelle
(1) R est un groupe 9-florényle ou aralkyle dont la partie alkyle contient un ou deux atomes de carbone et dont la partie aryle est un noyau aromatique ou hétéro-aromatique choisi entre phényle, furyle, thiényle, pyridyle et naphtyle, et ce noyau aromatique ou hétéro-aromatique est facultativement substitué avec un ou deux substituants choisis, indépendamment, entre le brome, le chlore, le fluor, un reste alkyle en C₁ à C₃ et un reste alkoxy en C₁ à C₃ ;
R⁶ et et R⁷ forment ensemble un groupe oxo ;
R⁸ est de l'hydrogène ;
R⁹ est un groupe COR¹ ;
R¹ est un groupe OR ou NHR³, dans lequel R est un reste alkyle en C₁ à C₃ et R³ est choisi entre furyle, thiényle, 2-thiazolyle, 2-benzothiazolyle, 3-méthyl-5-isothiazolyle et 5-phényl-1,3,4-thiazolyle ;
et R¹⁰ et R¹¹ représentent tous deux de l'hydrogène ; ou bien
(2) R est tel que défini ci-dessus ;
R⁶ est de l'hydrogène ;
R⁷ est un groupe CONHR³ dans lequel R³ est tel que défini ci-dessus ;
R⁸ et R⁹ forment ensemble un groupe oxo ; et
R¹⁰ et R¹¹ représentent tous deux de l'hydrogène ; ou bien
(3) R est de l'hydrogène ;
R⁶ et R⁷ forment ensemble un groupe oxo ;
R⁸ est de l'hydrogène ;
R⁹ est un groupe COR¹ dans lequel R¹ est tel que défini ci-dessus ;
R¹⁰ et R¹¹ forment, conjointement avec l'atome de carbone auquel ils sont attachés, un système bicyclique spiro-condensé, facultativement substitué, de formule : 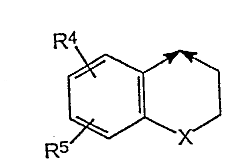
dans laquelle X est un groupe méthylène, de l'oxygène ou du soufre et R⁴ et R⁵ sont choisis, indépendamment, entre l'hydrogène, du brome, du chlore, du fluor et un reste alkoxy en C₁ à C₃ ; sous réserve que, lorsque R¹ est un reste éthoxy ou méthoxy, R représente autre chose qu'un reste benzyle ou benzyle substitué, dont les substituants sont des groupes 2-, 3- ou 4-chloro, 4-méthoxy ou 3,4-diméthoxy ; un reste phénéthyle ou phénéthyle substitué dont les substituants sont un groupe 4-chloro ou 4-méthyle ;
et les sels pharmaceutiquement acceptables de ce composé.

2. Composé suivant la revendication 1, de formule I dans laquelle les groupes substituants R et R¹ sont tels que définis dans la revendication 1

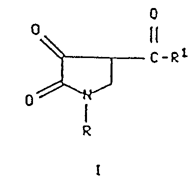

3. Composé suivant la revendication 1, répondant àla formule II dans laquelle les groupes substituants R et R³ sont tels que définis dans la revendication 1

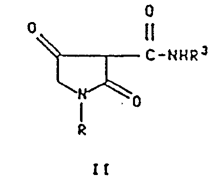

4. Composé suivant la revendication 1, de formule III dans laquelle les groupes substituants R¹, R⁴, R⁵ et X sont tels que définis dans la revendication 1

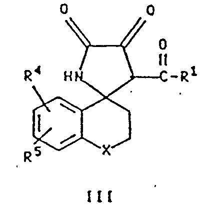

5. Composé suivant la revendication 2, dans lequel R¹ est un groupe éthoxy ou propoxy et R est un groupe 9-fluorényle ou aralkyle dont la partie alkyle est un groupe méthylène et la partie aryle est choisie entre phényle, naphtyle, 2-thiényle, 4-pyridyle, 2-pyridyle, 2-furyle, 4-bromophényle et 3,4-dichlorophényle.

6. Composé suivant la revendication 3, dans lequel R est un groupe aralkyle dont la partie alkyle est un groupe méthylène et la partie aryle est choisie entre phényle, 3,4-dichlorophényle et 4-méthoxyphényle et R³ est choisi entre les groupes 3-méthylisothiazole-3-yle, benzothiazole-2-yle, 5-méthylisothiazole-3-yle, thiazole-2-yle et 3-phényl-1,2,4-thiazole-5-yle.

7. Composé suivant la revendication 4, dans lequel R⁴ est un reste 6-fluoro, X est de l'oxygène et R¹ est un reste éthoxy.

8. Composé suivant la revendication 1, qui est choisi entre :
le 1-(thién-2-ylméthyl)-4,5-dioxopyrrolidine-3-carboxylate d'éthyle ;
le 1-(pyrid-2-ylméthyl)-4,5-dioxopyrrolidine-3-carboxylate d'éthyle ;
le 1-(pyrid-4-ylméthyl)-4,5-dioxopyrrolidine-3-carboxylate d'éthyle ;
le 1-(napht-1-ylméthyl)-4,5-dioxopyrrolidine-3-carboxylate d'éthyle ;
le 1-(4-bromobenzyl)-4,5-dioxopyrrolidine-3-carboxylate d'éthyle ;
le 1-(fur-2-ylméthyl)-4,5-dioxopyrrolidine-3-carboxylate d'éthyle ;
le 1-(3,4-dichlorobenzyl)-4,5-dioxopyrrolidine-3-carboxylate d'éthyle ;
le 1-(benzyl)-4,5-dioxopyrrolidine-3-carboxylate de propyle ;
le 1-(9-fluorényl)-4,5-dioxopyrrolidine-3-carboxylate d'éthyle ;
le N-(3-méthylisothiazole-5-yl)-1-benzyl-4,5-dioxopyrrolidine-3-carboxamide;
le N-(benzothiazole-2-yl)-1-benzyl-4,5-dioxopyrrolidine-3-carboxamide;
le N-(5-méthylisoxazole-3-yl)-1-benzyl-4,5-dioxopyrrolidine-3-carboxamide ;
le N-(thiazole-2-yl)-1-(3,4-dichlorobenzyl)-2,4-dioxopyrrolidine-3-carboxamide ;
le N-(benzothiazole-2-yl)-1-(3,4-dichlorobenzyl)-2,4-dioxopyrrolidine-3-carboxamide;
le N-(3-phényl-1,2,4-thiadiazole-5-yl)-1-(3,4-dichlorobenzyl)-2,4-dioxopyrrolidine-3-carboxamide ;
le N-(benzothiazole-2-yl)-1-(2-phényléthyl)-2,4-dioxopyrrolidine-3-carboxamide ;
le N-(3-méthylisothiazole-5-yl)-1-(4-méthoxybenzyl)-2,4-dioxopyrrolidine-3-carboxamide;
le N-(benzothiazole-2-yl)-1-(4-métholybenzyl)-2,4-dioxopyrrolidine-3-carboxamide; et
l'ester éthylique d'acide spiro-[4H-1-benzopyranne-4,2'-pyrrolidine]-3'-carboxylique, à substitution 6-fluoro-2,3-dihydro-4',5'-dioxo et les sels pharmaceutiquement acceptables de ce composé.

9. Composition pharmaceutique destinée à prévenir ou à atténuer une complication associée au diabète telle que neuropathie, néphropathie, rétinopathie et cataracte chez un mammifère diabétique, cette composition comprenant une quantité suffisante d'un composé suivant la revendication 1 efficace pour prévenir ou atténuer cette complication, et un support acceptable du point de vue pharmaceutique.

10. Procédé de préparation d'un composé de formule 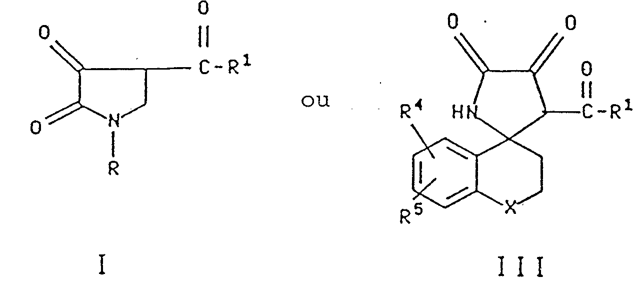 dans laquelle R, R¹, R⁴, R⁵ et X sont tels que définis dans la revendication 1, ou d'un sel pharmaceutiquement acceptable de ce composé, comprenant la réaction d'un composé de formule 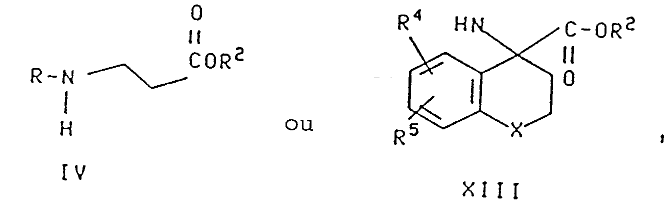 respectivement avec un composé de formule 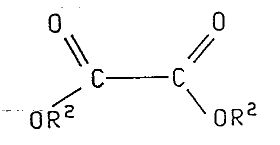 dans laquelle R, R, R⁴, R⁵ et X sont tels que définis ci-dessus, et, le cas échéant, la préparation d'un composé de formule I ou III dans laquelle R¹ est un groupe R³NH par réaction d'un composé de formule respective I ou III, dans laquelle R¹ est un groupe OR, avec un composé de formule R³NH₂ dans laquelle R³ est choisi entre les groupes furyle, thiényle, 2-thiazolyle, 2-benzothiazolyle, 3-méthyl-5isothiazolyle et 5-phényl-1,3,4-thiazolyle et la transformation facultative des composés de formule I ou III en sels pharmaceutiquement acceptables.

11. Procédé de production d'un composé de formule 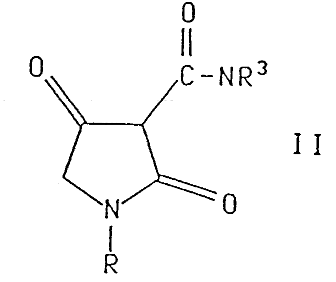 dans laquelle R et R³ sont tels que définis dans la revendication 1, comprenant a) le traitement d'un composé de formule 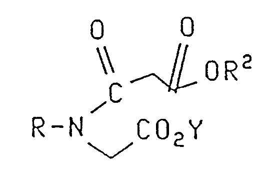 dans laquelle R et R sont tels que définis dans la revendication 1 et Y est un groupe alkyle en C₁ à C₃ avec un alcoolate alcalin ou alcalino-terreux convenable et b) la réaction du produit de l'étape a) avec un composé de formule R³NH₂ dans laquelle R³ est tel que défini ci-dessus ; et facultativement la transformation du produit de cette réaction en un sel pharmaceutiquement acceptable.

12. Procédé suivant la revendication 10, pour l'obtention d'un composé de formule I dans laquelle R¹ est un groupe éthoxy ou propoxy et R est choisi entre des groupes 9-fluorényle et aralkyle dont la partie alkyle est un groupe méthylène et la partie aryle est choisie entre phényle, naphtyle, 2-thiényle, 4-pyridyle, 2-pyridyle, 2-furyle et 4-bromophényle.

13. Procédé suivant la revendication 10, pour l'obtention d'un composé de formule III dans laquelle R¹ est un groupe éthoxy, R⁴ est un groupe 6-fluoro, R⁵ est de l'hydrogène et X est de l'oxygène.

14. Procédé suivant la revendication 10, pour l'obtention d'un composé de formule I dans laquelle R¹ est un groupe NHR³ et R, R³ sont tels que définis dans la revendication 1.

15. Procédé suivant la revendication 10, pour l'obtention d'un composé de formule II dans laquelle R est un groupe aralkyle dont la partie alkyle est un groupe méthylène et la partie aryle est choisie entre phényle, 3,4-dichlorophényle et 4-méthoxyphényle et R³ est choisi entre 3-méthylisothiazole-3-yle, benzothiazole-2-yle, 5-méthylisothiazole-3-yle, thiazole-2-yle et 3-phényl-1,2,4-thiazole-5-yle.

16. Procédé suivant la revendication 10, pour l'obtention d'un composé de formule I choisi entre
le 1-(thién-2-ylméthyl)-4,5-dioxopyrrolidine-3-carboxylate d'éthyle ;
le 1-(pyrid-2-ylméthyl)-4,5-dioxopyrrolidine-3-carboxylate d'éthyle ;
le 1-(pyrid-4-ylméthyl)-4,5-dioxopyrrolidine-3-carboxylate d'éthyle ;
le 1-(napht-1-ylméthyl)-4,5-dioxopyrrolidine-3-carboxylate d'éthyle ;
le 1-(4-bromobenzyl)-4,5-dioxopyrrolidine-3-carboxylate d'éthyle ;
le 1-(fur-2-ylméthyl)-4,5-dioxopyrrolidine-3-carboxylate d'éthyle ;
le 1- (3,4-dichlorobenzyl)-4,5-dioxopyrrolidine-3-carboxylate d'éthyle ;
le 1-(benzyl)-4,5-dioxopyrrolidine-3-carboxylate de propyle ;
le 1-(9-florényl)-4,5-dioxopyrrolidine-3-carboxylate d'éthyle ; et des sels pharmaceutiquement acceptables de ce composé.

17. Procédé suivant la revendication 10, pour l'obtention d'un composé de formule I choisi entre
le N-(3-méthylisothiazole-5-yl)-1-benzyl-4,5-dioxopyrrolidine-3-carboxamide ;
le N-(benzothiazole-2-yl)-1-benzyl-4,5-dioxopyrrolidine-3-carboxamide ;
le N-(5-méthylisoxazole-3-yl)-1-benzyl-4,5-dioxopyrrolidine-3-carboxamide ; et des sels pharmaceutiquement acceptables de ce composé.

18. Procédé suivant la revendication 10, pour l'obtention de l'ester éthylique d'acide spiro-[4H-1-benzopyranne-4,2'-pyrrolidine]-3'-carboxylique, à substitution 6-fluoro-2,3-dihydro-4',5'-dioxo ; et de ses sels pharmaceutiquement acceptables.

19. Procédé suivant la revendication 11, pour l'obtention d'un composé de formule II choisi entre
le N-(thiazole-2-yl)-1-(3,4-dichlorobenzyl)-2,4-dioxopyrrolidine-3-carboxamide ;
le N-(3-phényl-1,2,4-thiadiazole-5-yl)-1-(3,4-dichlorobenzyl)-2,4-dioxopyrrolidine-3-carboxamide ;
le N-(benzothiazole-2-yl)-1-(2-phényléthyl)-2,4-dioxopyrrolidine-3-carboxamide ;
le N-(3-méthylisothiazole-5-yl)-1-(4-méthoxybenzyl)-2,4-dioxopyrrolidine-3-carboxamide ;
le N-(benzothiazole-2-yl)-1-(4-méthoxybenzyl)-2,4-dioxopyrrolidine-3-carboxamide ; et des sels pharmaceuti-quement acceptables de ce composé.

## Revendications (Revendications pour l'(les) Etat(s) contractant(s) suivant(s): ES, GR)

1. Procédé de production d'un composé de formule 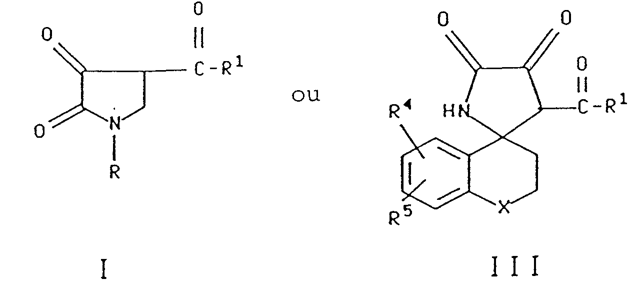 dans laquelle
R est un groupe 9-florényle ou aralkyle dont la partie alkyle contient un ou deux atomes de carbone et dont la partie aryle est un noyau aromatique ou hétéro-aromatique choisi entre phényle, furyle, thiényle, pyridyle et naphtyle, ce noyau aromatique ou hétéro-aromatique étant facultativement substitué avec un ou deux substituants choisis, indépendamment, entre le brome, le chlore, le fluor, des restes alkyle en C₁ à C₃ et des restes alkoxy en C₁ à C₃ ;
R¹ est un groupe OR ; dans lequel R est un reste alkyle en C₁ à C₃ ;
R⁴ et R⁵ sont choisis, indépendamment, entre l'hydrogène, le brome, le chlore, le fluor et un reste alkoxy en C₁ à C₃ ; et
X est un groupe méthylène, l'oxygène ou le soufre ;
sous réserve que, lorsque R¹ est un reste éthoxy ou méthoxy, R représente autre chose qu'un reste benzyle ou benzyle substitué, dont les substituants sont des groupes 2-, 3- ou 4-chloro, 4-méthoxy ou 3,4-diméthoxy ; un reste phénéthyle ou phénéthyle substitué dont les substituants sont un groupe 4-chloro ou 4-méthyle ;
ou d'un sel pharmaceutiquement acceptable de ce composé ; comprenant la réaction d'un composé de formule 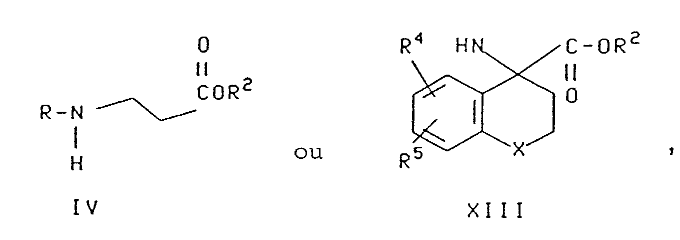 respectivement avec un composé de formule 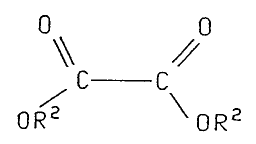 où R, R, R⁴, R⁵ et X sont tels que définis ci-dessus, et, le cas échéant, la préparation d'un composé de formule I ou III dans laquelle R¹ est un groupe R³NH par réaction d'un composé de formule respective I ou III, dans laquelle R¹ est un groupe OR, avec un composé de formule R³NH₂ dans laquelle R³ est choisi entre furyle, thiényle, 2-thiazolyle, 2-benzothiazolyle, 3-méthyl-5-isothiazolyle et 5-phényl-1,3,4-thiazolyle ; et la transformation facultative des composés de formule I ou III en sels pharmaceutiquement acceptables.

2. Procédé de production d'un composé de formule 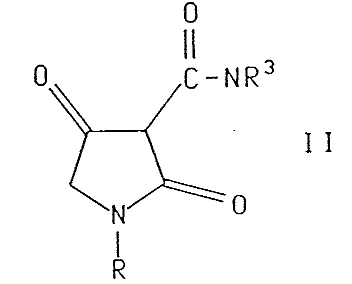 dans laquelle R et R³ sont tels que définis dans la revendication 1, comprenant a) le traitement d'un composé de formule 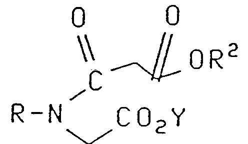 dans laquelle R et R sont tels que définis dans la revendication 1 et Y est un groupe alkyle en C₁ à C₃ avec un alcoolate alcalin ou alcalino-terreux convenable et b) la réaction du produit de l'étape a) avec un composé de formule R³NH₂ dans laquelle R³ est tel que défini ci-dessus ; et facultativement, la transformation du produit de cette réaction en un sel pharmaceutiquement acceptable.

3. Procédé suivant la revendication 1, pour l'obtention d'un composé de formule I dans laquelle R¹ est un reste éthoxy ou propoxy et R est choisi entre un reste 9-florényle ou aralkyle dont la partie alkyle est un groupe méthylène et la partie aryle est choisie entre phényle, naphtyle, 2-thiényle, 4-pyridyle, 2-pyridyle, 2-furyle et 4-bromophényle.

4. Procédé suivant la revendication 1, pour l'obtention d'un composé de formule III dans laquelle R¹ est un reste éthoxy, R⁴ est un reste 6-fluoro, R⁵ est de l'hydrogène et X est de l'oxygène.

5. Procédé suivant la revendication 1, pour l'obtention d'un composé de formule I dans laquelle R¹ est un groupe NHR³ et R, R³ sont tels que définis dans la revendication 1.

6. Procédé suivant la revendication 1, pour l'obtention d'un composé de formule II dans laquelle R est un groupe aralkyle dont la partie alkyle est un groupe méthylène et la partie aryle est choisie entre phényle, 3,4-dichlorophényle et 4-méthoxyphényle et R³ est choisi entre 3-méthylisothiazole-3-yle, benzothiazole-2-yle, 5-méthylisothiazole-3-yle, thiazole-2-yle et 3-phényl-1,2,4-thiazole-5-yle.

7. Procédé suivant la revendication 1, pour l'obtention d'un composé de formule I choisi entre
le 1-(thién-2-ylméthyl)-4,5-dioxopyrrolidine-3-carboxylate d'éthyle ;
le 1-(pyrid-2-ylméthyl)-4,5-dioxopyrrolidine-3-carboxylate d'éthyle ;
le 1-(pyrid-4-ylméthyl)-4,5-dioxopyrrolidine-3-carboxylate d'éthyle ;
le 1-(napht-1-ylméthyl)-4,5-dioxopyrrolidine-3-carboxylate d'éthyle ;
le 1-(4-bromobenzyl)-4,5-dioxopyrrolidine-3-carboxylate d'éthyle ;
le 1-(fur-2-ylméthyl)-4,5-dioxopyrrolidine-3-carboxylate d'éthyle ;
le 1-(3,4-dichlorobenzyl)-4,5-dioxopyrrolidine-3-carboxylate d'éthyle ;
le 1-(benzyl)-4,5-dioxopyrrolidine-3-carboxylate de propyle ;
le 1-(9-florényl)-4,5-dioxopyrrolidine-3-carboxylate d'éthyle ; et ses sels pharmaceutiquement acceptable.

8. Procédé suivant la revendication 5, pour l'obtention d'un composé de formule I choisi entre
le N-(3-méthylisothiazole-5-yl)-1-benzyl-4,5-dioxopyrrolidine-3-carboxamide ;
le N-(benzothiazole-2-yl)-1-benzyl-4,5-dioxopyrrolidine-3-carboxamide ;
le N-(5-méthylisoxazole-3-yl)-1-benzyl-4,5-dioxopyrrolidine-3-carboxamide ; et des sels pharmaceutiquement acceptables de ce composé.

9. Procédé suivant la revendication 4, pour l'obtention de l'ester éthylique d'acide spiro-[4H-1-benzopyranne-4,2'-pyrrolidine]-3'-carboxylique, à substitution 6-fluoro-2,3-dihydro-4',5'-dioxo ; et de ses sels pharmaceutiquement acceptables.

10. Procédé suivant la revendication 6, pour l'obtention d'un composé de formule II choisi entre
le N-(thiazole-2-yl)-1-(3,4-dichlorobenzyl)-2,4-dioxopyrrolidine-3-carboxamide ;
le N-(3-phényl-1,2,4-thiadiazole-5-yl)-1-(3,4-dichlorobenzyl)-2,4-dioxopyrrolidine-3-carboxamide ;
le N-(benzothiazole-2-yl)-1-(2-phénéthyl)-2,4-dioxopyrrolidine-3-carboxamide ;
le N-(3-méthylisothiazole-5-yl)-1-(4-méthoxybenzyl)-2,4-dioxopyrrolidine-3-carboxamide ;
le N-(benzothiazole-2-yl)-1-(4-méthoxybenzyl)-2,4-dioxopyrrolidine-3-carboxamide ; et de leurs sels pharmaceutiquement acceptables.
